(19) Europäisches Patentamt
European Patent Office
Office européen des brevets

(11) **EP 4 171 369 B1**

(12) **EUROPÄISCHE PATENTSCHRIFT**

(45) Veröffentlichungstag und Bekanntmachung des Hinweises auf die Patenterteilung:
**07.05.2025 Patentblatt 2025/19**

(21) Anmeldenummer: **21739285.1**

(22) Anmeldetag: **24.06.2021**

(51) Internationale Patentklassifikation (IPC):
*A61B 5/021* (2006.01)     *A61B 5/022* (2006.01)

(52) Gemeinsame Patentklassifikation (CPC):
**A61B 5/02108; A61B 5/022**

(86) Internationale Anmeldenummer:
**PCT/EP2021/067270**

(87) Internationale Veröffentlichungsnummer:
**WO 2021/260082 (30.12.2021 Gazette 2021/52)**

(54) **VERFAHREN ZUM BETREIBEN EINER BLUTDRUCKMESSVORRICHTUNG**

METHOD FOR OPERATING A BLOOD PRESSURE MEASURING APPARATUS

PROCÉDÉ DE FONCTIONNEMENT D'UN APPAREIL DE MESURE DE TENSION ARTÉRIELLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.06.2020 DE 102020116750**

(43) Veröffentlichungstag der Anmeldung:
**03.05.2023 Patentblatt 2023/18**

(73) Patentinhaber: **Redwave Medical GmbH**
**07745 Jena (DE)**

(72) Erfinder:
• **STOCKMANN, Chris**
  **07607 Eisenberg (DE)**
• **MAINKA, Andreas**
  **07747 Jena (DE)**
• **DITTRICH, Verena**
  **99084 Erfurt (DE)**

(74) Vertreter: **Meissner Bolte Partnerschaft mbB**
**Berliner Straße 1**
**07545 Gera (DE)**

(56) Entgegenhaltungen:
DE-A1- 102017 117 337     US-A1- 2003 069 490
US-A1- 2003 069 507       US-A1- 2016 150 983
US-A1- 2018 263 513

**Beschreibung**

Die Erfindung betrifft ein Verfahren zum Betreiben einer Blutdruckmessvorrichtung nach Anspruch 1 und eine Vorrichtung zum Ausführen des Verfahrens mit den Merkmalen des Anspruchs 11 sowie einer Verwendung des Verfahrens und der Vorrichtung nach Anspruch 16.

Oszillometrisch arbeitende Blutdruckmessvorrichtungen sind bekannt. Diese werden in der Regel so betrieben, dass zunächst eine Manschette um eine Extremität, z.B. Oberarm, gelegt wird. Die Manschette wird mittels einer Pumpe aufgepumpt. Durch den Druck der Manschette wird der Blutfluss in einem innerhalb der Extremität verlaufenden Gefäß unterbrochen. Der Druck in der Manschette wird sodann wieder abgelassen, sodass sich der Blutfluss in dem abgedrückten Gefäß wieder nachweisen lässt. Dabei ergibt sich der Blutdruck in diesem Blutgefäß durch den momentan in der Manschette herrschenden Druck. Mit einem solchen Verfahren lassen sich beispielsweise der mittlere arterielle Druck und daraus sowohl der systolische als auch der diastolische Blutdruck im Blutgefäß ermitteln. Insbesondere die Absenkungen des Blutdrucks in der Nacht beim Schlaf trägt eine wichtige Rolle bei der Erkennung und Therapie von Bluthochdruck oder Stress.

Der Stand der Technik zeigt auf, dass zur nichtinvasiven Bestimmung des Blutdrucks eine Manschette an einer Extremität appliziert werden muss. Durch das Aufpumpen dieser Manschette von mind. 20 mmHg über dem erwarteten systolischen Blutdruck wird an der Extremität des Anwenders mind. ein Druck von über 140 mmHg (oft auch 180 mmHg) ausgeübt. Dies ist nicht nur unangenehm, sondern kann unter Umständen schmerzhaft sein. Zusätzlich wird bei Nachtmessungen der Schlaf erheblich gestört, wodurch die natürliche Absenkung des Blutdrucks beeinflusst und das Ergebnis damit verfälscht wird. Aufgrund der erforderlichen Ablassgeschwindigkeit des Drucks in der Manschette von <7 mmHg/Sek dauert eine solche Blutdruckmessung zwischen 30 Sek bis 90 Sek.

Abwandlungen dieser Systeme können den Blutdruck anhand der Signale während der Aufpumpphase ermitteln. Diese benötigen jedoch einen ähnlich hohen Zieldruck im System von bspw. 20 mmHg über der erwarteten Systole. Auch die Aufpumpgeschwindigkeit sollte 7 mmHg/Sek nicht überschreiten. Damit bleiben die wesentlichen Nachteile dieser Technologie erhalten.

Der systolische und diastolische Blutdruck sind wesentliche Parameter, mit denen sich der Kreislaufzustand eines Patienten oder Anwenders charakterisieren lässt. Diese Parameter reichen jedoch in vielen Fällen nicht aus. Sehr häufig lassen sich detaillierte Aussagen über die Hämodynamik eines Lebewesens nur dann treffen, wenn eine genauere Kenntnis der Gefäßeigenschaften vorliegt. Diese lassen sich aus einer einfachen Blutdruckmessung der bekannten Art nicht gewinnen, sondern dafür ist eine Analyse der Pulswellen notwendig.

Aus dem Stand der Technik bekannte Systeme, welche Messungen ohne Manschetten ermöglichen (bspw. über optische Sensoren oder Piezosensoren, wie bei der Tonometrie), üben zwar weniger Stress auf den Anwender aus, sie sind jedoch nicht in der Lage valide Blutdruckwerte zu ermitteln. Sie erfordern eine Kalibrierung der Messwerte vor jeder einzelnen Messung. Diese sind die Grundlage für die Transformation, welche zur Bestimmung des Blutdrucks verwendet wird. Die vorgelagerte Manschetten-Messung ist somit weiterhin erforderlich und definiert die Genauigkeit des Systems.

Weitere bekannte Geräte zur Pulswellenanalyse steuern gezielt einen konstanten Druck an, ein sogenanntes Druck-Plateau, welches für mehrere Sekunden, üblicherweise 10 Sekunden, gehalten wird. Auch diese Methode ist allein nicht in der Lage den Blutdruck zu bestimmen und erfordert stets eine direkt vorgelagerte Standardmessung als Referenz.

Ein weiteres sehr großes Problem von Systemen, welche derzeit versuchen den Blutdruck ohne Manschette zu bestimmen, ist, dass bei der Durchführung einer Pulswellenanalyse eine einzelne Formel zur Transformation (generelle Transferfunktion) für die gesamte Population eingesetzt wird, womit eine deutliche Unschärfe und Qualitätseinbußen einhergehen. Darüber hinaus wird für die Kalibrierung nur ein Referenzblutdruck angegeben. Verfahren zum Betreiben einer Blutdruckmessvorrichtung, welche ebenfalls die Pulswelle bei konstantem Druck mit der Messmanschette aufzeichnen, sind aus US2018/263513 oder US2003/069507 bekannt.

Es besteht daher die Aufgabe, ein Verfahren anzugeben, mit dem der Blutdruck ohne den bekannten Vorgang des Aufpumpens und Ablassens einer Blutdruckmanschette ermittelt werden kann. Im konkreten soll ein Zieldruck von 100 mmHg nicht überschritten werden, wobei das gesuchte Verfahren eine valide Blutdruckmessung und eine Pulswellenanalyse mit zusätzlichen hämodynamischen Parametern ermöglichen soll. Damit soll es ermöglicht werden, die Qualität der Messdaten während einer Spotmessung, einer Langzeitmessung und einer Nachtmessung zu verbessern, indem der Tragekomfort erhöht, der Stress auf den Anwender reduziert, die Messdauer einer Einzelmessung verkürzt und die Anzahl der Messungen je Messreihe erhöht werden kann.

Die Lösung der Aufgabe erfolgt mit einem Verfahren zum Betreiben einer Blutdruckmessvorrichtung mit den Merkmalen des Anspruchs 1.

Das Verfahren zum Betreiben einer Blutdruckmessvorrichtung beinhaltet folgende Verfahrensschritte:

- Applizieren einer Messvorrichtung, enthaltend eine Druckmesseinheit und/oder eine Druckeinheit an einer Messstelle am Körper,
- Initialisieren der Messvorrichtung mit einem Ausführen einer Referenzmessung und/ oder durch ein Ausführen definierter Lageveränderungen der Messstelle und Bestimmen personenspezifischer Initialisierungsparameter,

- Speichern der personenspezifischen Initialisierungsparameter in einer Speicher- und Steuereinheit,

- Ausführen mindestens einer Blutdruckmessung bei Vorliegen eines Gegendrucks durch die Druckeinheit in einem subsystolischen Niedrigdruckbereich,

- Beibehalten des vorliegenden Gegendrucks innerhalb einer Niedrigdruck-Plateauphase mit einer vorbestimmten Zeitdauer und Registrieren von zeitlichen Blutdruckverlaufsdaten während der vorbestimmten Zeitdauer,

- Umrechnen der registrierten zeitlichen Blutdruckverlaufsdaten über die Initialisierungsparameter in zeitliche arterielle Blutdruckdaten, wobei. das Initialisieren durch Ausführen der Referenzmessung mit folgenden Schritten erfolgt:

  - Anlegen eines Messdrucks an die Messvorrichtung in einem definierten Druckbereich,
  - Definiertes Ändern, bspw. Ablassen, des Messdrucks aus der Messvorrichtung mit einem Registrieren des zeitlichen Druckverlaufs während des Vorgangs in der Steuer- und Speichereinheit,
  - Extraktion eines oszillierenden Pulsanteils aus dem zeitlichen Druckverlauf während des Vorgangs in Verbindung mit einer Speicherung einer Reihe von Daten zu einzelnen Pulswellen in der Steuer- und Speichereinheit,
  - Signalanalyse der einzelnen Pulswellen und Datenabgleich mit einem gegebenen Pulswellensignalmodell durch die Steuer- und Speichereinheit,
  - Ermitteln einer personenspezifischen Übertragungsfunktion aus dem Datenabgleich und Speichern der ermittelten Übertragungsfunktion als personenspezifischer Initialisierungsparameter in der Steuer- und Speichereinheit, wobei weiterhin nach der Initialisierung und der Bestimmung der Koeffizienten der Übertragungsfunktion mit einer oszillometrischen Niedrigdruck-Plateaumessung und der zum konstanten Plateau-Druck zugehörigen Übertragungsfunktion $H_k(j\omega)$ aus einem oszillierenden Signalanteil der Niedrigdruck-Plateaumessung die arterielle Pulswelle berechnet wird und die Signalanalyse der einzelnen Pulswellen mit folgenden Schritten erfolgt:

    - Auswertung der Form der jeweiligen Pulswelle und Klassifizieren der jeweiligen Pulswelle in einer Auswerteeinheit und Ablegen in einem internen Speicher,
    - Zusammenfügen und Transformieren von Pulswellen aus mindestens einer Klassifizierung zu mindestens einem, die Merkmale der arteriellen Pulswelle abbildenden Pulswellensignalmodell,

- Anpassen der gemessenen Pulswellenverläufe an das mindestens eine Pulswellensignalmodell und Ermitteln mindestens einer Übertragungsfunktion für das jeweilige Pulswellensignalmodell.

Gegebenenfalls wird bei einer vorteilhaften Ausgestaltung des Verfahrens eine Validierung der Übertragungsfunktion ausgeführt, wobei bei der Blutdruckmessung bei einem gegebenen Gegendruck ermittelte und gespeicherte Blutdruckverlaufsdaten mit gegebenen Referenzparametern verglichen werden.

Insbesondere bei der Blutdruckmessung im subsystolischen Niedrigdruckbereich erfolgt bei einer vorteilhaften Verfahrensgestaltung in der Steuer- und Speichereinheit eine Umrechnung der gemessenen zeitlichen Blutdruckverlaufsdaten über eine invertierte, aus dem Initialisierungsschritt bestimmte personenspezifische Übertragungsfunktion in arterielle Blutdruckwerte.

Bei der Blutdruckmessung im subsystolischen Niedrigdruckbereich in der Steuerund Speichereinheit wird bei einer Gestaltung des Verfahrens eine iterierte modellbasierte Blutdruckbestimmung ausgeführt, wobei über eine Signaltransformation eine abweichungsminimierende Anpassung von Parametern eines Pulswellensignalmodells an den oszillierenden Signalanteil des gemessenen Blutdrucks erfolgt.

Bei der Validierung der Initialisierungsparameter wird bei einer Ausgestaltung die modellbasierte Blutdruckbestimmung im subsystolischen Niedrigdruckbereich mit dem Pulswellensignalmodell und einem Signalverlauf aus der Referenzmessung verglichen und das dabei bestimmbare Initialisierungsergebnis mit den vorhandenen Initialisierungsparametern verglichen.

Bei einer Gestaltung erfolgt die Referenzmessung durch eine in dem Blutgefäß befindliche katheterartige Blutdruckmessvorrichtung, wobei der dabei ermittelte Zeitverlauf des Blutdrucks mit einem parallel dazu ermittelten zeitlichen Druckverlauf an der Messvorrichtung abgeglichen und dabei die Übertragungsfunktion bestimmt wird.

Für die Initialisierung und/oder die Blutdruckmessung kann als Druckeinheit der Messvorrichtung eine aufpumpbare Druckmanschette oder eine Kombination aus einer Druckmanschette und einem einen konstanten subsystolischen Druck ausübenden Bekleidungsstück verwendet werden.

Als Druckeinheit der Messvorrichtung kann eine aufpumpbare Druckmanschette in Kombination mit einem optischen Sensor verwendet werden, wobei die aufpumpbare Druckmanschette für das Ausführen der Referenzmessung und der optische Sensor für die Blutdruckmessung im subsystolischen Niedrigdruckbereich verwendet wird.

Eine Vorrichtung zum Ausführen eines Verfahrens, welche nicht Bestandteil der vorliegenden Erfindung ist, umfasst folgende Komponenten:

Vorgesehen ist eine Steuer- und Speichereinheit mit einer Anzeige, einem internen Initialisierungsprogramm und einem Steuerprogramm, einem Speicher, einem Prozessor und Bus sowie einem Plateau-Generator, einer von der Steuerund Speichereinheit gesteuerten Initialisierungs- und Messeinheit mit einem Drucksensor und einem Druckaktuator.

Die Steuer- und Speichereinheit enthält vorteilhaft einen Digitalen Signalverarbeitungsprozessor.

Die Steuer- und Speichereinheit weist bei einer vorteilhaften Gestaltung eine Kommunikationseinheit für einen Datenaustausch über ein externes Kommunikationsnetz auf.

Bei einer vorteilhaften Ausgestaltung ist eine externe Auswerteeinheit vorgesehen, wobei diese über eine Geräteschnittstelle mit der Steuer- und Speichereinheit koppelbar ist.

Die externe Auswerteeinheit kann zweckmäßigerweise eine Anzeige, ein Konfigurationsprogramm und/oder ein Auswerteprogramm und/oder eine Benutzerschnittstelle aufweisen.

Das genannte Verfahren und die genannte Vorrichtung nach einem der vorhergehenden Ausführungsformen ist zum Ermitteln von Blutdruckparametern, insbesondere Systole, Diastole und weiteren hämodynamischen Parametern mittels einer Pulswellenanalyse vorgesehen.

Das Verfahren und die Vorrichtung sollen nachfolgend anhand beispielhafter Ausführungsformen und Verfahrensabläufe näher beschrieben werden. Zur Verdeutlichung dienen die beigefügten Figuren 1 bis 28. Es werden für gleiche oder gleich wirkende Teile dieselben Bezugszeichen verwendet.

Es zeigt:

Fig. 1    einen beispielhaften Aufbau einer Blutdruckmessvorrichtung,

Fig. 2    eine vereinfachte Darstellung des Übertragungsweges der vom Herzen erzeugten Pulswelle von der Quelle bis zum Messsensor exemplarisch für eine oszillometrische Messung,

Fig. 3    eine Darstellung eines für die Niedrigdruck-Blutdruckbestimmung dominierenden Übertragungsgliedes (links) und einer daraus abgeleitete parametrisierte Übertragungsfunktion (rechts),

Fig. 4    eine Darstellung des beispielhaften Anwendungsfalls A1,

Fig. 5    einen beispielhaften Ablauf eines 24-Blutdruck-Monitorings bei niedrigem Gegendruck mit einem mobilen Blutdruckmessgerät,

Fig. 6    eine Darstellung eines beispielhaften Anwendungsfalls A2,

Fig. 7    eine Darstellung eines beispielhaften Anwendungsfalls B,

Fig. 8    eine Darstellung eines beispielhaften Anwendungsfalls C1,

Fig. 9    eine Darstellung eines beispielhaften Anwendungsfalls C2,

Fig. 10   eine Darstellung beispielhafter Schnittstellen der Initialisierung des Verfahrens und notwendige Vorverarbeitungsschritte vor der Initialisierung,

Fig. 11   eine detaillierte Darstellung beispielhafter Schnittstellen der Initialisierung des Verfahrens und notwendige Vorverarbeitungsschritte vor der Initialisierung bei Verwendung einer oszillometrischen Messung zur Initialisierung,

Fig. 12   eine beispielhafte oszillometrische Messung mit Blutdruckmanschette mit vollständigem Aufpumpvorgang über den zu erwartenden systolischen Blutdruck gefolgt von einem Ablassvorgang als Referenzmessung für die Initialisierung,

Fig. 13   Oszillierender Signalanteil $p_{osc}(t)$ aus einem vollständigen Ablassvorgang einer oszillometrischen Messung,

Fig. 14   Ablauf der Pulswellenanalyse zur Extraktion von Pulswellenmerkmalen und zur Bestimmung einer personenspezifischen Pulswellenvorlage (PW-Vorlage),

Fig. 15   An den Pulswellen-Fußpunkten ausgerichteter oszillierender Signalanteil $p_{osc}(t)$ für das Beispiel aus Fig. 12,

Fig. 16   Pulswellenvorlage mit beispielhaften Pulswellenmerkmalen, die aus dem Tangentenschnittpunkt-Verfahren (Strichlinien) hervorgehen,

Fig. 17   Beispiel für eine aus dem suprasystolischen Bereich des oszillometrischen Signalanteils einer Manschettenmessung bestimmten Pulswellenvorlage (links), Rechts: mittelwertbereinigte, gemittelte Pulswelle einer invasiven Blutdruckmessung mittels Katheter aus der A. brachialis im selben Patienten, gegenüberliegender Arm,

Fig. 18    Beispiel für ein aus verketteten und skalierten PW-Vorlagen rekonstruiertes Pulswellensignalmodell (PW-Signalmodell),

Fig. 19    einen Ausschnitt aus Fig. 18,

Fig. 20    beispielhaftes, aus dem PW-Signalmodell durch Skalierung mit den Blutdruckwerten $P_{SYS}$ und $P_{DIA}$ berechnetes PW-Signal als Eingangsvariable für die Initialisierung,

Fig. 21    beispielhafter Ablauf der iterativen Initialisierung zur Bestimmung von personenspezifischen Koeffizienten für die das Übertragungsverhalten nachbildende mathematische Abbildungsvorschrift,

Fig. 22    ein beispielhaftes Ergebnis nach erfolgreicher Initialisierung,

Fig. 23    ein Beispiel einer im modellbasierten Ansatz verwendeten Druck-Volumenbeziehung zur Berechnung von oszillometrischen Messdaten anhand des vorgegebenen Manschettendrucks und eines arteriellen PW-Signalmodells,

Fig. 24    beispielhafte Verfahrensschritte zur Berechnung der Modellantwort als wesentlicher Bestandteil der Initialisierung und Durchführung der modellbasierten Blutdruckbestimmung,

Fig. 25    beispielhafte oszillometrische Niedrigdruck-Plateaumessung mit Blutdruckmanschette,

Fig. 26    ein Beispiel für eine iterative modellbasierte Blutdruckbestimmung,

Fig. 27    beispielhafte Eingangs- und Ausgangsparameter für die Validierung des Initialisierungsergebnisses. Messablauf = Messkonfiguration,

Fig. 28    beispielhafte Eingangs- und Ausgangsparameter zur Bewertung des Initialisierungsergebnisses mit individueller Konfiguration einer beliebigen Anzahl von Niedrigdruckmessungen zum Blutdruck-Monitoring.

Das zur Ausführung des erfindungsgemäßen Verfahrens verwendete System ist möglichst einfach und robust gestaltet, so dass dieses Verfahren mit niedrigem Gegendruck der breiten Masse der niedergelassenen Ärzte aber auch den Patienten zuhause einfach und kostengünstig zur Verfügung gestellt werden kann.

Das Verfahren zum Betreiben einer Blutdruckmessvorrichtung umfasst bei allen genannten Beispielen folgende Verfahrensschritte:

Es erfolgt als erstes eine Applikation einer Messvorrichtung mit einem definierten Druck an einer Messstelle des Körpers des Patienten oder Anwenders, wobei die Messvorrichtung einen Sensor zum Anbringen an einer Messstelle, eine Befestigungseinheit und eine Druckregeleinheit zum Befestigen des Sensors mit einem definierten Druck umfasst.

In einem nächsten Schritt erfolgt das Initialisieren der Messvorrichtung durch Referenzmessungen und/oder definierte Manöver durch den Anwender, dabei werden personenspezifische Parameter aus der Initialisierung bestimmt.

Es erfolgt darauf eine Generierung individueller Algorithmen auf Grundlage der personenspezifischen Parameter, ein Registrieren und Speichern des zeitlichen Signalverlaufs am Sensor in einer Steuer- und Speichereinheit und eine Signalanalyse des Signals innerhalb der Steuer- und Speichereinheit.

Das Verfahren zum Betreiben einer Blutdruckmessvorrichtung erfolgt insbesondere mit folgenden Verfahrensschritten:

Es erfolgt eine Applikation einer Messvorrichtung mit einem definierten Druck an einer Messstelle, wobei die Messvorrichtung einen Sensor zum Anbringen an einer Messtelle, eine Befestigungseinheit und eine Druckregeleinheit zum Befestigen des Sensors mit einem definierten Druck umfasst.

Es erfolgt dann ein Initialisieren der Messvorrichtung durch Referenzmessungen und/oder definierte Manöver des Patienten oder Anwenders. Diese Manöver beinhalten insbesondere definierte Änderungen der Körperlage des Patienten oder Anwenders und/oder der Haltung einzelner Körperteile des Patienten oder Anwenders. Dabei erfolgt ein Registrieren und Speichern des zeitlichen Signalverlaufs am Sensor in einer Steuer- und Speichereinheit.

Es erfolgt dann die Bestimmung von personenspezifischen Parametern aus der Initialisierung mit den folgenden Schritten:

Es erfolgt zuerst ein Zerlegen eines pulsartigen Signalanteils in Daten über Einzelperioden zum Identifizieren von einzelnen Pulswellen.

Danach wird Extraktion von Features, d.h. von Formmerkmalen in den Pulswellen ausgeführt. Dies erfolgt durch einen Vergleich der Pulswellen aus der Referenzmessung mit den veränderten Signalen durch die vorerwähnten definierten Manöver des Patienten oder Anwenders.

In Verbindung damit wird ein Klassifizieren der einzelnen Pulswellen mit den jeweiligen identifizierten Features ausgeführt.

Es erfolgt ein Transformieren dieser Features in geeignete Input-Parameter für die jeweiligen Algorithmen und ein Hinzufügen zu einer Datenbank.

Es erfolgt dabei die Generierung individueller Algorithmen auf Grundlage der patientenspezifischen Parameter, wobei folgende Schritte ausgeführt werden:
Ein Registrieren und Speichern des zeitlichen Signalverlaufs am Sensor in einer Steuer- und Speichereinheit und eine Signalanalyse des Signals wird innerhalb der Steuer- und Speichereinheit ausgeführt.

Bei der Signalanalyse werden folgende Schritte ausgeführt:

Es erfolgt ein Zerlegen des pulsartigen Signalanteils in Daten über Einzelperioden zum Identifizieren von einzelnen Pulswellen.

Anschließend erfolgt eine Extraktion von Features in den Pulswellen. Dem schließt sich eine Bewertung der extrahierten Features durch Vergleich mit aus einer Datenbank bekannten Signale und Features an.

Es erfolgt dann in einem weiteren Schritt ein Auswerten der bewerteten Features zum Ermitteln eines Blutdrucks (wie bspw. brachialer Blutdruck, zentraler Blutdruck) und weiterer hämodynamischer Parameter (wie bspw. Pulswellengeschwindigkeit, Vascular Aging, Cardiac Output, ...).

Die Applikation der Messvorrichtung kann zum Beispiel durch eine Manschette, ein Pflaster, ein Armband, einen Clip und/oder ähnliches erfolgen. Für die Auswahl des Sensors sind neben einer Manschette insbesondere optische (PPG), elektromagnetische (z.B. Nahfeld-Radar) sowie mechanoelektrische Sensoren (wie Piezosensoren, Dehnungssensoren) und weitere (druckempfindliche) Sensoren möglich. Die Messstelle kann sich praktisch an einer beliebigen Stelle am Körper des Patienten oder Anwenders befinden (bspw. am Handgelenk, Unterarm, Oberarm, Brust, Hals, Stirn, Ohr, usw.). Der niedrige Gegendruck hat einen definierten Wert und befindet sich konstant zum Beispiel zwischen 0 mmHg und 100 mmHg.

Es können durch definierte Manöver gezielt Veränderungen im Signal hervorgerufen werden, um auf dieser Grundlage personenspezifische Parameter zu bestimmen.

Diese Parameter sind dann beispielsweise dazu geeignet, um Koeffizienten, Startwerte, Erwartungswerte und Grenzen zu bestimmen, die für den Patienten oder Anwender spezifisch sind.

Zur Generierung individueller Algorithmen auf Grundlage der personenspezifischen Parameter werden folgende Schritte ausgeführt:

Es erfolgt das Erstellen einer Datenbank von geeigneten, parametrisierbaren Algorithmen. Es erfolgt danach eine Auswahl klassenspezifischer Algorithmen auf Grundlage von ermittelten Features. Schließlich erfolgt ein Setzen der entsprechenden Parameter als Eingabe für die jeweiligen Algorithmen.

Das genannte Verfahren wird zum Ermitteln und Analysieren von Pulswellen, zum Bestimmen eines Blutdrucks und weiterer hämodynamischer Parameter verwendet.

Die Erfindung betrifft in den nachfolgen genannten Ausführungsbeispielen ein Verfahren zum Betreiben einer Blutdruckmessvorrichtung, sowie die Verwendung eines derartigen Verfahrens zum Ermitteln und analysieren von Pulswellen.

Das Verfahren erfolgt beispielhaft mit folgenden Verfahrensschritten und Mitteln:

Durchführen einer BPM (Blutdruckmessung) mit niedrigem Gegendruck auf die Messstelle. Dabei wird der Sensor wird auf die Haut gesetzt und mit niedrigem, definiertem Druck fixiert (0 < P <100 mmHg; bspw. 10 mmHg, 60 mmHg, 90 mmHg). Dabei kann die Dauer bis zum Erreichen des Zieldrucks für die Messung reduziert werden, bspw. auf 0 Sek bei dauerhaftem Gegendruck oder auf 5 Sek, bei einem kurzzeitigen Aufbau des Gegendrucks. Als Messdauer auf dem Zieldruck wird beispielsweise ein Zeitintervall von bis zu 30 Sek gewählt.

Als mögliche Sensoren kann beispielsweise ein so genannter optisch arbeitender Sensor zur Anwendung kommen. Es wird dabei ein Photoplethysmogramm (PPG) mit verschiedenen Wellenlängen z.B. grün, rot, infrarot möglich aufgenommen.

Möglich ist auch die Verwendung eines piezoelektrischen oder elektromagnetischen Sensors. Ebenso kann die aus der herkömmlichen Oszillometrie bekannte Konfiguration aus aufpumpbarer Manschette und Luft sowie eine Direktmessung mittels Tonometrie erfolgen.

Angewandt werden können auch Dehnungssensoren. Eine durch Pulsation veränderter Druck resultiert in einer Dehnung, welche zu Spannungsänderungen und damit Pulswellen führt. Ggf. können EKG-Trigger als optionale Unterstützung mit zur Anwendung kommen.

Die Messpositionen sind grundsätzlich frei, beispielsweise können Finger, Handgelenk, Unterarm, Oberarm, Oberkörper, Brust, Hals, Stirn und/oder Ohr zur Anwendung kommen.

Zu bestimmende Parameter sind der Blutdruck (systolisch, MAP, diastolisch, lokal (z.B. brachial) und zentral sowie weitere Hämodynamische Parameter (Pulswellengeschwindigkeit, Vascular Aging, Cardiac Output/EF/SV).

Es können hierzu folgende beispielhafte Schritte umgesetzt werden:

Zum Erheben der Rohdaten wird der Sensor an der ausgewählten Messstelle appliziert. Es werden dann Pulswellen über einen Zeitraum mehrerer Sekunden aufgenommen und gespeichert.

Ein definierter Druck auf die Sensoreinheit verhindert dabei eine MorphologieÄnderung der Pulswellen, die durch äußere Einwirkungen verursacht wird.

Eine Initialisierung der Messvorrichtung ist erforderlich und kann dabei durch besondere Manöver des Patienten oder Anwenders ausgeführt werden, wie zum Beispiel durch Lage- und Positionsveränderungen wie zum Beispiel in Form eines Übergangs von einer liegenden nach eine sitzenden Haltung, eine Bewegung des Arms nach unten/mittel/nach oben zeigend, durch Veränderungen der Herzfrequenz von Ruhe (z.B. 75/min) zu Belastung (z.B. >120/min), durch Atembefehle (Hecheln, tief und langsam), durch Sprechen (Text vorlesen) sowie durch Laufen.

Ggf. können auch Referenzwerte aus Standard-Blutdruckmessungen verwendet werden. Das System lernt dadurch personenspezifisch die Morphologie-Änderungen, welche durch Manöver verursacht werden (da der Druck von außen konstant bleibt). Derartige Initialisierungen sollten regelmäßig wiederholt werden (z.B. 1x/Jahr). Dies schafft einen Bezugsrahmen für eine Signalanalyse, die angepasst an ist an den Sensor, die Messstelle und/oder das Druckniveau.

Sensor-Fusion kann dabei vorteilhaft sein. Dazu lassen sich beispielsweise ein Piezo-Sensor mit einem PPG-Sensor und einem EKG kombinieren.

Abhängigkeiten sind dabei gegebenenfalls zu berücksichtigen, wie zum Beispiel die Herzfrequenz, das Alter, das Geschlecht, der Zustand als Raucher oder Nichtraucher, eine sonstige Medikation sowie Vorerkrankungen, wie Broken-Heart-Syndrom, Vorhofflimmern (AFib) oder Herzinsuffizienz.

Die Signalanalyse erfolgt dadurch, dass eine Liste von Algorithmen gegeben ist, welche durch individuelle Eingaben an personenspezifische Gegebenheiten angepasst werden können. Dadurch wird berücksichtigt, dass nicht bei jedem Patienten oder Anwender alle Algorithmen mit gleicher Initialisierung und Gewichtung zum Einsatz kommen.

Die Kalibrierwerte liefern somit für die Signalanalyse: Koeffizienten, Startwerte, Erwartungswerte. Es werden Abgrenzungen von richtig/falsch ermöglicht sowie eine Erkennung von Falsch-Positiv/Falsch-Negativ.

Für jeden Patienten oder Anwender wird demnach ein individuelles Set an Algorithmen generiert. Grundlage dafür sind die Informationen aus der Initialisierung.

Dazu kommen ausgewählte, bekannte oder neue Modelle, bspw. DruckBestimmung nach Windkessel-Modell und das Ermitteln von peripherem Widerstand R und Compliance C im Pulswellensignal zur Anwendung. Die Compliance C ist dabei die reziproke Elastizität.

Bei Anwendung des Reservoir-Excess-Modells erfolgt eine Abschätzung des Blutflusses.

Bei Anwendung des Korteweg-Modells wird die Abhängigkeit des Blutdrucks von der Pulswellengeschwindigkeit ermittelt. Die Ermittlung der Pulswellengeschwindigkeit erfolgt durch Wellenzerlegung der Pulswelle in Forward-Backward-Waves. Hier können so genannte Regression Trees bzw. ein Neuronales Netz zur Anwendung kommen.

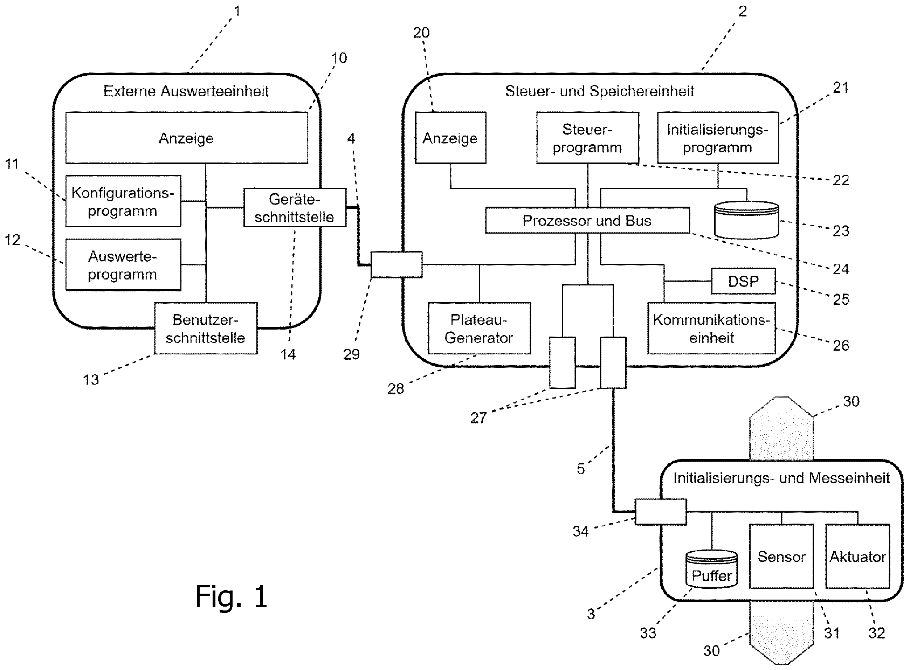

Fig. 1 zeigt einen beispielhaften Aufbau einer Blutdruckmessvorrichtung. Die Blutdruckmessvorrichtung enthält eine Externe Auswerteeinheit 1 mit einer Anzeige 10 und einem Konfigurationsprogramm 11 sowie einem Auswerteprogramm 12. Es ist weiterhin eine Benutzerschnittstelle 13 und eine Geräteschnittstelle 14 vorgesehen. Die Benutzerschnittstelle 13 ist beispielsweise eine Tastatur oder ein entsprechendes Bedienfeld, die Geräteschnittstelle ermöglicht einen Datenaustausch mit externen Geräten.

Die externe Auswerteeinheit kann beispielsweise ein ferner Server, aber auch ein lokal verfügbares Endgerät mit einer entsprechenden App sein. Die Geräteschnittstelle kann eine Schnittstelle für deine drahtlose oder drahtgebundene Datenübertragung sein, beispielsweise eine Bluetooth-Verbindung zur Kommunikation mit dem Endgerät, eine USB-Verbindung oder auch eine Verbindung über ein Kommunikationsnetz sein. In jedem Fall ermöglicht die externe Auswerteeinheit eine Fernaufzeichnung und Fernauswertung der registrierten Blutdruckmessdaten. Die externe Auswerteeinheit 1 kann sich beispielsweise in einer Facharztpraxis in Form eines Computers befinden, die eine Fernabfrage über den Zustand eines entfernten Patienten oder Anwenders ausführt.

Zur eigentlichen Ausführung sämtlicher Blutdruckmessungen ist eine Steuer- und Speichereinheit 2 vorgesehen. Diese enthält eine Anzeige 20 und einen Speicher mit einem darauf ablaufenden Initialisierungsprogramm 21 und einem Steuerprogramm 22. Zum Ausführen der Programme ist ein Speicher 23 und gegebenenfalls zusätzlich ein Prozessor mit Bus 24 vorgesehen. Zusätzlich kann ein Digitaler Signalverarbeitungsprozessor 25 vorgesehen sein, der grundlegende Signalverarbeitungsroutinen ausführt, eine Kommunikationseinheit 26 kann außerdem vorgesehen sein, über die die beispielsweise Sprachein- und -ausgaben erfolgen können. Die Steuer- und Auswerteeinheit enthält weiterhin eine Schnittstelle 27 zur Messeinheit und/oder zu einer Initialisierungs- und Messeinheit 3. Es können mehrere Messeinheiten angeschlossen werden um bspw. ein Sensor-Fusion herzustellen. Außerdem ist eine Schnittstelle 29 zur externen Auswerteeinheit 1 vorgesehen.

Zum Registrieren der Blutdruckwerte am Körper des Patienten oder Anwenders und/oder zum Initialisieren ist eine Initialisierungs- und Messeinheit 3 vorgesehen. Die Initialisierungs- und Messeinheit 3 kann somit sowohl zur Initialisierung als auch zur eigentlichen Messung verwendet werden. Möglich sind aber auch Gestaltungen, bei der die Initialisierung einerseits und die Messung andererseits von verschieden gestalteten Einheiten ausgeführt werden.

Die hier beispielhaft gezeigte Initialisierungs- und Messeinheit 3 weist eine Befestigungsvorrichtung 30 zur Fixierung der Messeinheit an der Messstelle des Patienten oder Anwenders, beispielsweise einen Gurt, auf. Zur Erfassung des Drucks dient ein Sensor 31, zur Beaufschlagung der Messstelle mit Druck dient ein Aktuator 32. Der Aktuator kann auf verschiedene Weise ausgebildet sein, beispielsweise als ein elektromechanischer Aktuator bei manschettenloser Messung, oder eine Pumpe bei einer Messanordnung mit einer Manschette.

Zweckmäßigerweise ist ein Puffer 33 zum kurzzeitigen Zwischenspeichern der Messdaten vor der Datenübertragung sowie eine Schnittstelle 34 zur Steuer- und Auswerteeinheit vorgesehen.

Die Daten werden jeweils über eine kabelgebundene oder kabellose Schnittstellenverbindung 4 und eine Schnittstellenverbindung 5, die ebenfalls kabelgebunden oder kabellos ausgeführt sein kann, übertragen und ausgetauscht.

Mögliche beispielhafte Ausprägungen des Messsensors 31 sind insbesondere ein akustischer Sensor mit entsprechenden internen Komponenten, ein optischer Sensor, insbesondere ein so genannter PPG-Sensor, ein elektromagnetischer, ein tonometrischer Messsensor oder auch ein Pflaster oder Kompressionsstrumpf mit integrierten Dehnungssensoren, der einen konstanten Druck auf die entsprechende Messposition ausübt. Als Aktuator 32 kommt beispielsweise eine Blutdruckmanschette oder auch ein Kompressionsstrumpf in Betracht. Der Kompressionsstrumpf bietet den Vorteil einer Dehnungsmessung und einer Kompression gleichermaßen. Er kann somit als Sensor und Aktuator gleichermaßen dienen.

Mögliche beispielhafte Messpositionen sind beispielsweise der Bereich des Oberarms, der Unterarm oder das Handgelenk, der Oberschenkel, der Unterschenkel oder das Fußgelenk. Möglich ist aber auch eine Messung im Ohrkanal oder am Ohrläppchen, an der Stirn, am Hals oder im Bereich des Thorax.

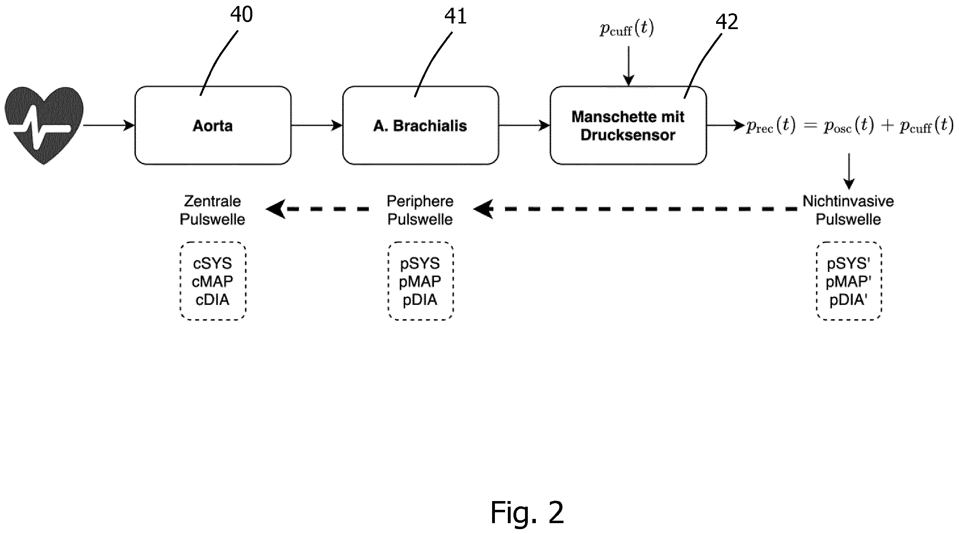

Fig. 2 zeigt eine vereinfachte Darstellung des Übertragungsweges der vom Herzen erzeugten Pulswelle von der Quelle bis zum Messsensor exemplarisch für eine oszillometrische Messung.

Das Verfahren, aus dem nichtinvasiv aufgenommenen oszillierenden Messsignal $p_{osc}(t)$ Rückschlüsse auf die arterielle Pulswelle (sowohl peripher als auch zentral) und ihre Eigenschaften zu ziehen, stellt sich messtechnisch als Lösung eines sogenannten inversen Problems dar. Dabei soll aus den Beobachtungen, hier dem Messsignal, die zugrundeliegende Quelle bzw. deren Eigenschaften bestimmt werden.

Fig. 2 zeigt in stark vereinfachter Darstellung die Übertragungsabschnitte der gemessenen Pulswelle. Diese breitet sich von ihrer Quelle, dem Herzen (bzw. linker Ventrikel), über die Aorta 40 hin zum Ort der peripheren Messung an der A. brachialis 41 (d.h. der Oberarmarterie) aus. Bei der Verwendung einer oszillometrischen Messung mit einer Manschette 42 erfolgt eine Erfassung des oszillatorischen Anteils $p_{osc}$ im Aufpump- und/oder Ablassvorgang des aufgezeichneten Manschettendrucks $p_{rec}$.

Um aus den gemessenen Druckdaten $p_{rec}$ und insbesondere aus dem dabei gemessenen oszillatorischen Anteil $p_{osc}$ auf die tatsächlichen Druckverhältnisse $p_{brach}$ in der Arterie rückschließen zu können, müssen die Übertragungseigenschaften bekannt sein, unter denen sich der tatsächliche arterielle Druck $p_{brach}$ in den gemessenen Druck $p_{osc}$ unter dem Einfluss des Manschettengegendrucks $p_{cuff}$ übersetzt. Vor dem eigentlichen Messvorgang ist daher eine Initialisierung der Messanordnung notwendig, um die Übertragungseigenschaften möglichst genau zu erfassen, zu modellieren und für die nachfolgenden regulären Messungen verfügbar zu machen. Die damit verbundenen Signalverarbeitungen sollen im Folgenden erläutert werden.

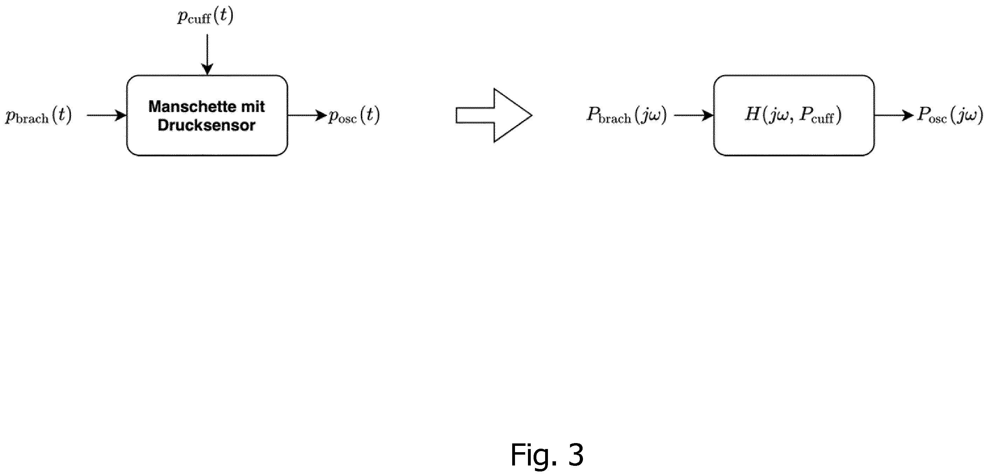

Fig. 3 zeigt hierzu eine Darstellung eines für die Niedrigdruck-Blutdruckbestimmung dominierenden Übertragungsgliedes im linken Teilbild und rechts eine daraus abgeleitete parametrisierte Übertragungsfunktion $H(j\omega, P_{cuff})$, die den arteriellen Druck $P_{brach}(j\omega)$ in den gemessenen oszillatorischen Druck $P_{osc}(j\omega)$ überführt.

Die Betrachtung dieser Übertragungseigenschaften für die oszillometrische Messung kann also näherungsweise als eine Übertragungsfunktion mit dem Manschettengegendruck $P_{cuff}$ als Parameter betrachtet werden, die die arterielle Pulswelle in der A. brachialis und das gemessene Pulssignal (d.h. die oszillierende Komponente von $p_{rec}$) zueinander in Beziehung setzt:

$$P_{osc}(j\omega) = P_{brach}(j\omega)H(j\omega, P_{cuff})$$

Unter der vereinfachten Annahme von Zeitinvarianz der Übertragungseigenschaften für die Dauer von einer Pulswelle kann nun bei Kenntnis der Übertragungsfunktion $H(j\omega, P_{cuff})$ und dem zugehörigen, konstanten Manschettengegendruck $P_{cuff}$ die arterielle Pulswelle aus der gemessenen Pulswelle bestimmt werden.

$$P_{brach}(j\omega) = \frac{P_{osc}(j\omega)}{H_k(j\omega)}$$

Dabei ist $H_k(j\omega)$ die zum Manschettengegendruck $P_{cuff}$ zugehörige Übertragungsfunktion. Es besteht somit die Aufgabe für einen vorgegebenen Gegendruck die zugehörige Übertragungsfunktion

$$H_k(j\omega) = \frac{P_{osc}(j\omega)}{P_{brach}(j\omega)}$$

zu bestimmen.

Bei der Betrachtung mehrerer, in einem vorgegebenen Zeitraum stattfindenden, Blutdruckmessungen (z.B. während eines 24h-Monitorings), kann es notwendig sein, auch zwischen den Messungen auftretenden personenspezifischen Änderungen der Übertragungseigenschaften zu berücksichtigen. Dazu kann die Übertragungsfunktion $H_k(j\omega)$ vereinfacht in zwei Bestandteile gegliedert werden. Dies ist zum einen eine personenspezifische über den Messzeitraum invariante Übertragungsfunktion $H_{k,filt}(j\omega)$, die von den Filtereigenschaften des die Arterie umgebenden Gewebes und der Manschette dominiert wird. Zum anderen kann eine personenspezifische zeitvariante Übertragungsfunktion, $H_{k,hemo}(j\omega)$ angesetzt werden, die von den sich zwischen den Messzeitpunkten ändernden hämodynamischen Eigenschaften des Patienten oder Anwenders (z.B. Arteriensteifigkeit, Arterienkontraktion, etc.) dominiert wird. $H_{k,hemo}(j\omega)$ kann zudem diagnostisch relevante Informationen enthalten, die sich in einer bestimmten Änderung der Pulswellenmorphologie und Pulswellenmerkmale widerspiegelt. So kann $H_{k,hemo}(j\omega)$ beispielsweise die auftretenden personenspezifischen Unterschiede zwischen zwei Blutdruckmessungen charakterisieren, die jeweils am Anfang und am Ende eines Tages aufgezeichnet wurden.

Die Bestimmung der druckabhängigen Übertragungsfunktion $H(j\omega, P_{cuff})$ bzw. einer daraus abgeleiteten Menge von zu konstanten Gegendrücken $P_{cuff}$ zugeordneten Übertragungsfunktionen $H_k(j\omega)$ erfolgt durch eine personenspezifische Initialisierung des Verfahrens.

Nach der Initialisierung kann eine Blutdruckmessung mit einem konstanten, subsystolischen Gegendruck $P_{cuff}$ zwischen 0 und 100 mmHg durchgeführt werden, um aus dem gemessenen oszillierenden Signalanteil $P_{osc}(j\omega)$ und der aus der Initialisierung bekannten Übertragungsfunktion $H_k(j\omega)$ $P_{brach}(j\omega)$ und somit die gesuchten Blutdruckwerte zu ermitteln. Infolge des niedrigen Gegendrucks wird die Patientenbelastung erheblich reduziert und eine mögliche Beeinflussung der Blutdruckwerte, beispielsweise bei einer Nachtmessung, vermieden.

Nachfolgend soll das Verfahren anhand einzelner Anwendungsfälle dargestellt werden.

Anwendungsfall A1

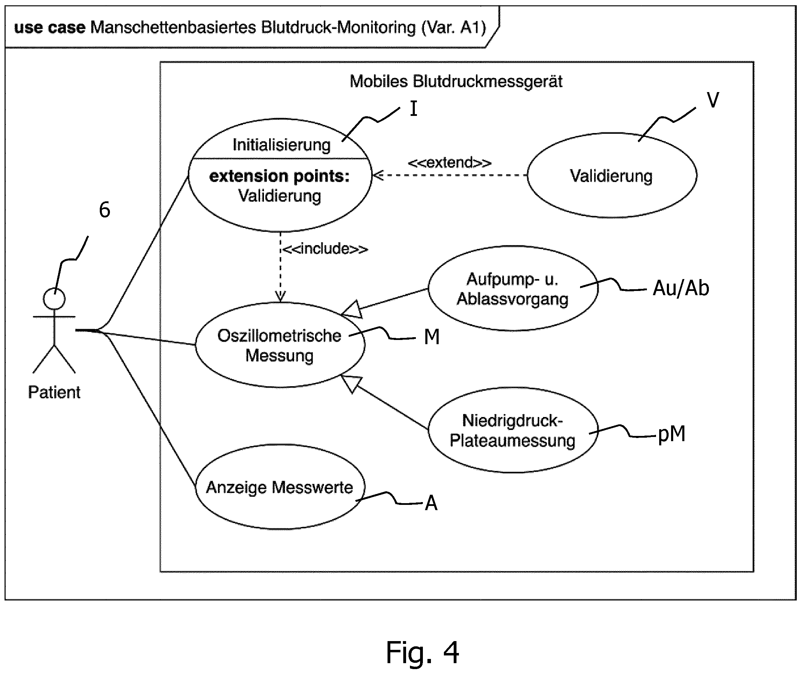

Der Anwendungsfall A1 ist in Fig. 4 als Übersicht dargestellt. Das Ausführungsbeispiel des Verfahrens nach Anwendungsfall A1 kann in Verbindung mit einem oszillometrischen, manschettenbasierten Blutdruckmessgerät zum Monitoring des Patientenblutdrucks im Homecare-Einsatz verwendet werden. Dabei wird die Manschette kontinuierlich über den gesamten Messzeitraum, insbesondere während einer 24-Stunden-Messung vom Patienten/Anwender 6 getragen.

In Anwendungsfall A1 werden eine Initialisierung I, eine oszillometrische Messung M und eine Auswertung der Messsignale und Vitalparameter autonom durch das Blutdruckmessgerät durchgeführt und durch den Patienten oder Anwender initiiert. Gegebenenfalls kann außerdem eine Validierung V ausgeführt werden, bei der die Resultate der Initialisierung überprüft werden. Es erfolgt natürlich eine Anzeige A der Messwerte.

Nach der Initialisierung I des Verfahrens werden während des Messzeitraums zum Blutdruck-Monitoring in fest definierten Abständen Niedrigdruck-Plateaumessungen pM durchgeführt, bei denen die Manschette auf einen konstanten Manschettendruck aufgepumpt wird, der deutlich unter dem zu erwartenden systolischen Blutdruck liegt.

Zu Beginn des Messzeitraums, d.h. zu Beginn des Monitorings, erfolgt die Bestimmung der personenspezifischen, vom Manschettendruck abhängigen Übertragungsfunktionen über die Initialisierung I des Verfahrens. In einer ersten Ausführung des Verfahrens wird zur Initialisierung eine vollständige oszillometrische Messung M durchgeführt, bei der der Manschettendruck auf einen Spitzenwert gepumpt wird, welcher mindestens 20 mmHg über den zu erwartenden systolischen Blutdruck liegt.

Aus der vollständigen oszillometrischen Messung M wird der oszillierende Signalanteil, $p_{osc}$, des Manschettendrucks während der Aufpump- und/oder Ablassvorgänge Au/Ab extrahiert. Aus dem Amplitudenverlauf von $p_{osc}$ erfolgt eine Bestimmung des mittleren arteriellen Blutdrucks ($P_{MAP}$) sowie des diastolischen ($P_{DIA}$) und systolischen ($P_{SYS}$) Blutdrucks.

Die personen- und gerätespezifischen Übertragungsfunktionen zur Charakterisierung der Filtereigenschaften des Übertragungsgliedes zwischen der peripheren Arterie, z.B. der A. brachialis, und dem Messsensor werden in der Initialisierung I des Verfahrens mithilfe des oszillierenden Drucksignals $p_{osc}$, des nichtoszillierenden Manschettendrucks $p_{cuff}$ (entspricht dem von der Manschette erzeugten Gegendruck) sowie der Blutdruckparameter $P_{DIA}$, $P_{MAP}$ und $P_{SYS}$ bestimmt.

Aus dem oszillierenden Drucksignal $p_{osc}$ werden dazu individuelle Pulswellen und personenspezifische Parameter durch eine Pulswellenwellenanalyse extrahiert. Anhand der die Pulswellenmorphologie und -dynamik beschreibenden Parameter werden die extrahierten Pulswellen klassifiziert und aus einer Teilmenge geeigneter Pulswellen eine Pulswellenvorlage bestimmt, die die Morphologie der arteriellen Pulswelle charakterisiert.

Aus der Pulswellenvorlage wird nun ein auf den Zeitverlauf des gemessenen oszillierenden Drucksignals $p_{osc}$ angepasstes Pulswellensignalmodell gebildet. Dabei werden insbesondere die durch die Hämodynamik beeinflussbaren Signaleigenschaften, bspw. der Pulswellenabstand, sowie Merkmale des Herzrhythmus, bspw. Extrasystolen oder kompensatorische Pausen, berücksichtigt. Unter Berücksichtigung der bereits bestimmten Blutdruckparameter kann das Pulswellensignalmodell in ein Pulswellensignal transformiert werden (z.B. durch Skalierung), welches die arterielle Pulswelle möglichst genau approximiert.

Mit dem aus dem Pulswellenmodell bestimmten Pulswellensignal PWSig und den aus der Messung bekannten oszillierenden und nichtoszillierenden Manschettendruckzeitverläufen ($p_{osc}$ und $P_{cuff}$) ist eine Bestimmung der Koeffizienten der personenspezifischen Übertragungsfunktionen $H_k$ möglich. Diese Koeffizientenbestimmung stellt dabei ein Optimierungsproblem dar, bei dem die Antwort des durch $H_k$ nachgebildeten Übertragungsgliedes einen möglichst minimalen Fehler zum Referenzsignal aufweisen soll. In der Initialisierung sind das Pulswellensignal und der nichtoszillierende Manschettendruck $P_{cuff}$ die Eingangssignale und der oszillierende Manschettendruck $p_{osc}$ das Referenzsignal.

Zusätzlich kann die Initialisierung weitere Parameter berücksichtigen, wie beispielsweise die Informationen über den Sensor, den Patienten oder Anwender sowie den Zustand des Messverfahrens. Beispielhaft sind hier die Manschettengröße als ein möglicher Sensorparameter, das Geschlecht, Alter, Größe und Gewicht als mögliche Anwender- oder Patientenparameter sowie vorherige Initialisierungsergebnisse als mögliche Zustandsparameter zu nennen.

Die in der Initialisierung ermittelten Koeffizienten zur Beschreibung des personenund gerätespezifischen Übertragungsverhaltens werden in einer Speichereinheit des Blutdruckmessgeräts gespeichert.

Mit den aus der Initialisierung bekannten Koeffizienten kann die Übertragungsfunktion $H_k$ für einen konstanten Manschettendruck $P_{cuff}$ oder für einen zeitveränderlichen Manschettendruck $p_{cuff}(t)$ bestimmt werden. Dadurch ist es möglich, anhand des oszillierenden Signalanteils $p_{osc}(t)$ aus einer Niedrigdruck-Plateaumessung mit konstantem Manschettendruck das arterielle Pulswellensignal zu bestimmen. Diese Bestimmung kann bei Dimensionierung der Koeffizienten für die Beschreibung der Übertragungsfunktion im Frequenzbereich durch eine Multiplikation von $H_k(j\omega)$ mit $P_{osc}(j\omega)$ erfolgen. Alternativ ist eine Nachbildung des Übertragungsverhaltens mit einem zeitdiskreten Differentialgleichungssystem empfehlenswert, welches die Lösung direkt im Zeitbereich durch eine iterative Ausgleichsrechnung ermöglicht. Die Lösung im Zeitbereich erlaubt im Gegensatz zum Frequenzbereich die Berücksichtigung nichtstationärer und zeitvarianter Effekte im Pulswellensignal.

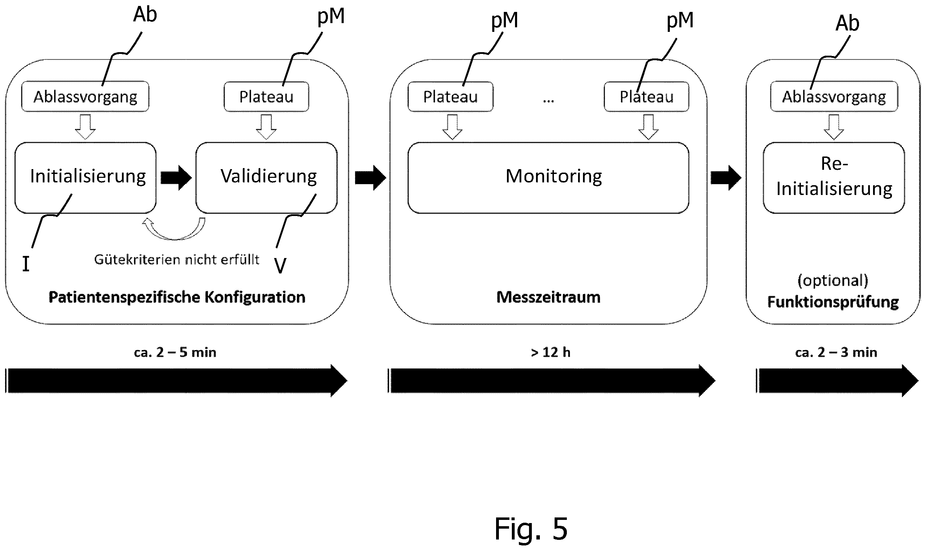

Fig. 5 zeigt die beispielhafte Durchführung des Verfahrens zur Blutdruckbestimmung bei niedrigem Gegendruck von der Initialisierung der Blutdruckmessvorrichtung bis zum ersten Schritt der Re-Initialisierung im Rahmen einer Funktionsprüfung.

In einem der Initialisierung I nachfolgenden Schritt der Validierung V werden die ermittelten und gespeicherten Koeffizienten anhand der Rekonstruktion des arteriellen Pulswellensignals und der Bestimmung der Blutdruckwerte validiert. Ziel der Validierung V ist eine Festlegung und/oder Validierung der Manschettendrücke für die eigentlichen Niedrigdruck-Plateaumessungen, die während des Messzeitraums durchgeführt werden sollen. So soll in der Validierung V beispielsweise ermittelt werden, ob ein vorher definierter konstanter Gegendruck für die Niedrigdruck-Plateaumessungen für den Patienten/Anwender geeignet ist, oder ob dieser noch etwas erhöht werden muss um ein zuverlässiges Pulswellensignal im oszillierenden Signalanteil der Manschette messen zu können.

In der Validierung V werden mithilfe der in der Initialisierung bestimmten manschettendruckabhängigen Übertragungskoeffizienten die Blutdruckparameter ($P_{DIA}$, $P_{MAP}$, $P_{SYS}$) aus dem oszillierenden Manschettendruck $p_{osc}$ bestimmt und mit den vom Blutdruckmessgerät aus dem Aufpump- oder Ablassvorgang Au/Ab ermittelten Werten, den Referenzwerten, verglichen. Die Validierung V ist erfolgreich, wenn die mit den Übertragungskoeffizienten ermittelten Blutdruckwerte eine vorher festgelegte Abweichung von den Referenzwerten nicht überschreiten. Bei ungültiger Validierung V erfolgt eine Wiederholung der Initialisierung I mit einer Wiederholung der Initialisierungsmessung, bspw. der vollständigen oszillometrischen Messung M. Ursachen für das Fehlschlagen der Initialisierung I können u. a. starke Störungen der zur Initialisierung verwendeten Messdaten infolge von Bewegungsartefakten oder fehlerhafter Sensorposition sein.

Eine geeignete Anpassung des Validierungsschrittes V ist die ersatzweise oder zusätzliche Bestimmung der Blutdruckparameter ($P_{DIA}$, $P_{MAP}$, $P_{SYS}$) anhand einer aus dem oszillierenden Manschettendruck $p_{osc}$ rekonstruierten Niedrigdruck-Plateaumessung pM. Dabei werden mithilfe der Pulswellenanalyse geeignete Pulswellen aus dem Ablassvorgang extrahiert und anschließend für einen aus dem nichtoszillierenden Manschettendruck bestimmten Gegendruck in eine virtuelle Niedrigdruck-Plateaumessung durch Signalrekonstruktion transformiert.

Der Validierungsschritt V kann erweitert werden, indem mehrere virtuelle Niedrigdruck-Plateaumessungen pM aus dem oszillierenden Druckanteil $p_{osc}$ rekonstruiert werden. Für jede rekonstruierte Niedrigdruck-Plateaumessung pM wird eine Bewertung durch eine eigene Validierung V gemäß der oben genannten Vorgehensweise durchgeführt und anhand der virtuellen Niedrigdruck-Plateaumessung mit der höchsten Güte werden die Messparameter für das Niedrigdruck-Monitoring bestimmt und in einer Niedrigdruck-Konfiguration gespeichert. Diese Niedrigdruck-Konfiguration ist insbesondere personenspezifisch und individualisiert. Beispielhafte Parameter, die in der Niedrigdruck-Konfiguration gespeichert werden können, sind der für die Messung benötigte Manschettendruck, die optimale Plateau-Dauer sowie die optimale Anzahl von Niedrigdruck-Plateaus.

Eine vorteilhafte Erweiterung der Validierung V ist die Kombination mehrerer virtueller Niedrigdruck-Plateaumessungen zur simultanen Bestimmung der Blutdruckparameter ($P_{DIA}$, $P_{MAP}$, $P_{SYS}$).

Eine geeignete Anpassung der Validierung V ist die ersatzweise oder zusätzliche Durchführung von realen Niedrigdruck-Plateaumessungen pM sowie Kombinationen von Niedrigdruck-Plateaumessungen pM zu entweder fest vorgegebenen oder aus mindestens einer vorhergehenden Initialisierungsmessung abgeleiteten niedrigen Gegendrücken zwischen 0 und 100 mmHg.

Nach erfolgreicher Validierung V werden das Initialisierungsergebnis, das Validierungsergebnis und die zum Blutdruck-Monitoring notwendigen Parameter strukturiert und in der externen oder internen Steuer- und Auswerteeinheit 1 und/oder 2 gespeichert. Notwendige Parameter sind beispielsweise durch die Vorgabe der Messintervalle, der Grenzwerte für die Berechnungsalgorithmen und insbesondere die Niedrigdruck-Konfiguration gegeben. Letztere definiert die zur Blutdruckmessung verwendeten Messparameter wie Gegendruck und Plateaudauer.

Im Messzeitraum wird das Blutdruck-Monitoring durchgeführt. Dabei werden in festdefinierten Zeitabständen eine oder mehrere Niedrigdruck-Plateaumessungen pM aufgezeichnet und die Blutdruckparameter mithilfe der in der Initialisierung ermittelten Übertragungskoeffizienten bestimmt.

Das Verfahren kann um eine autonome Funktionsprüfung erweitert werden, bei der mithilfe einer vollständigen oszillometrischen Messung M eine Re-Initialisierung durchgeführt und die ermittelten Übertragungskoeffizienten mit den in vorherigen Initialisierungs- bzw. Re-Initialisierungsschritten bestimmten Übertragungskoeffizienten verglichen wird.

Eine sinnvolle Erweiterung des Verfahrens ist die ersatzweise oder zusätzliche Funktionsprüfung bzw. Prüfung der Gültigkeit der Initialisierungsparameter während des Messzeitraums anhand der oszillierenden Signalkomponenten der Niedrigdruck-Plateaumessungen.

Anwendungsfall A2

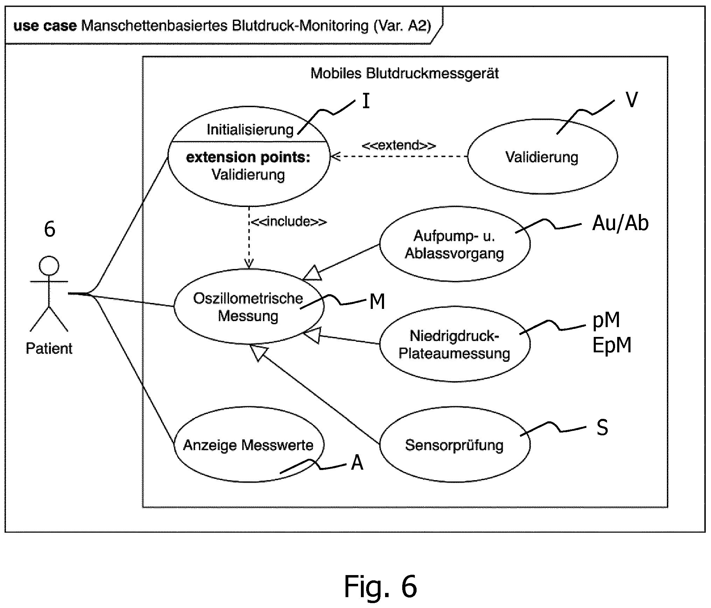

Eine sinnvolle Erweiterung des Messverfahrens stellt in Fig. 6 gezeigte Anwendungsfall A2 dar.

Bei dem Anwendungsfall A2 ist die tägliche Frequenz der Monitoring-Aufzeichnungen auf ein bis zwei Messungen reduziert, dafür wird aber eine längere Beobachtungszeit im Homecare-Einsatz von mehreren Tagen oder Wochen angestrebt. Da über einen längeren Beobachtungszeitraum je nach eingesetzter Sensortechnologie keine kontinuierliche Sensorpositionierung bzw. Sensorapplikation möglich ist, beispielsweise bei der oszillometrischen Messung mit einer Blutdruckmanschette, muss eine Prüfung der vom Sensor abhängigen Initialisierungsparameter vor einer Niedrigdruck-Plateaumessung durchgeführt werden.

Die in einem Abstand von beispielsweise 8 bis 10 Stunden durchgeführten Niedrigdruck-Plateaumessungen pM werden nachfolgend auch als Einzelpunkt-Niedrigdruck-Plateaumessung EpM (Single-Spot Niedrigdruck-Plateaumessung) bezeichnet. Vor und/oder während der Durchführung der Einzelpunkt-Niedrigdruck-Plateaumessung EpM wird eine Sensorprüfung S durchgeführt.

Die Sensorprüfung S ermöglicht über die Interaktion mit dem Anwender über eine Benutzerschnittstelle eine Korrektur der Sensorposition, bspw. des Manschettensitzes. Zudem ermöglicht die Sensorprüfung S die Anpassung einer Teilmenge der in der Initialisierung I bestimmten Übertragungskoeffizienten, insbesondere der Teilübertragungsfunktion $H_{k,filt}$, um die Genauigkeit der Blutdruckbestimmung aufrechtzuerhalten.

Die Sensorprüfung S kann beispielhaft durch die Analyse zusätzlicher multimodaler Sensor- und Aktordaten erfolgen, die während der Einzelpunkt-Niedrigdruck-Plateaumessung erfasst und mit aus der Initialisierung bestimmten Referenz- und Grenzwerten verglichen werden (z.B. Manschettenvolumen bei vorgegebenem niedrigem Gegendruck). Eine vorteilhafte Ausprägung des Verfahrens bestimmt die korrekte Applikation des Sensors anhand der aus einer Niedrigdruck-Plateaumessung extrahierten oszillierenden und nichtoszillierenden Signalkomponenten. Dabei werden beispielhaft Evaluierungsparameter aus einer Zeitreihenanalyse, Spektralanalyse und/oder Zeit-Frequenz-Verbundrepräsentation der Komponenten extrahiert und mit den aus der Initialisierung bestimmten Referenz- und Grenzwerten verglichen. Die Evaluierungsparameter können z.B. Trends und Steigungen des nichtoszillierenden Signalanteils sowie transiente Signale (Sprünge, Artefakte) in den Signalkomponenten charakterisieren.

Eine beispielhafte Ausführung des Anwendungsfalls A2 liegt darin, eine manschettenbasierte Niedrigdruck-Blutdruckbestimmung zu ermöglichen, bei der zwei Mal täglich (z.B. morgens und abends) je eine Niedrigdruck-Plateaumessung oder eine Kombination von Niedrigdruck-Plateaumessungen nach Neuanlegen der Blutdruckmanschette durchgeführt wird, ohne dass eine Re-Initialisierung des Verfahrens erforderlich ist.

Anwendungsfall B

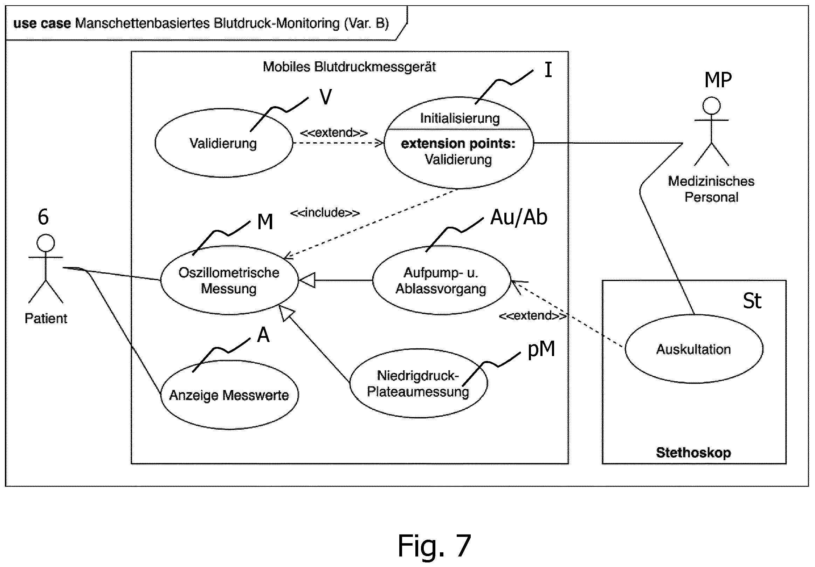

Fig. 7 zeigt eine mögliche Erweiterung der Anwendungsfälle A1 und A2. Dieser ist als Anwendungsfall B bezeichnet. Bei diesem Anwendungsfall wird das Blutdruck-Monitoring über einen eng begrenzten Zeitraum (z.B. 24-h-Monitoring) mit einem professionellen mobilen Blutdruck-Monitor durchgeführt.

In Anwendungsfall B wird die Initialisierung I bei einem Patientenbesuch in einer medizinischen Einrichtung (z.B. einer Arztpraxis) von professionellem medizinischem Personal MP vorgenommen. Dabei können zur Initialisierung die aus einer oszillometrischen Messung M mit einer Manschette ermittelten Referenzwerte (z.B. $P_{DIA}$, $P_{SYS}$) verwendet werden oder, wie in Fig. 7 dargestellt, anderweitig bestimmte Referenzwerte durch das medizinische Personal zugewiesen werden. Zusätzlich kann das medizinische Personal eine Überprüfung und Anpassung der Initialisierung (z.B. durch visuelle Aufbereitung mit einer grafischen Benutzeroberfläche), eine Kontrolle der in der Initialisierung ermittelten Parameter und die Eingabe zusätzlicher personenspezifischen Parameter durchführen.

Die Bestimmung der Referenzwerte kann z.B. durch den nach gegenwärtigem Stand der Technik akzeptierten Goldstandard der Auskultation St der Korotkow-Töne erfolgen. Dabei kann die Auskultation St vom Arzt selber vorgenommen werden oder durch das Blutdruckmessgerät unterstützt werden, z.B. durch die akustische Erfassung der Korotkow-Töne mithilfe eines elektronischen Stethoskops. Die Initialisierung I ist dann gültig für einen vorgegebenen Messzeitraum, z.B. 24 Stunden.

Die Messhäufigkeit beträgt ca. alle 15 - 30 Minuten.

Anwendungsfall C1

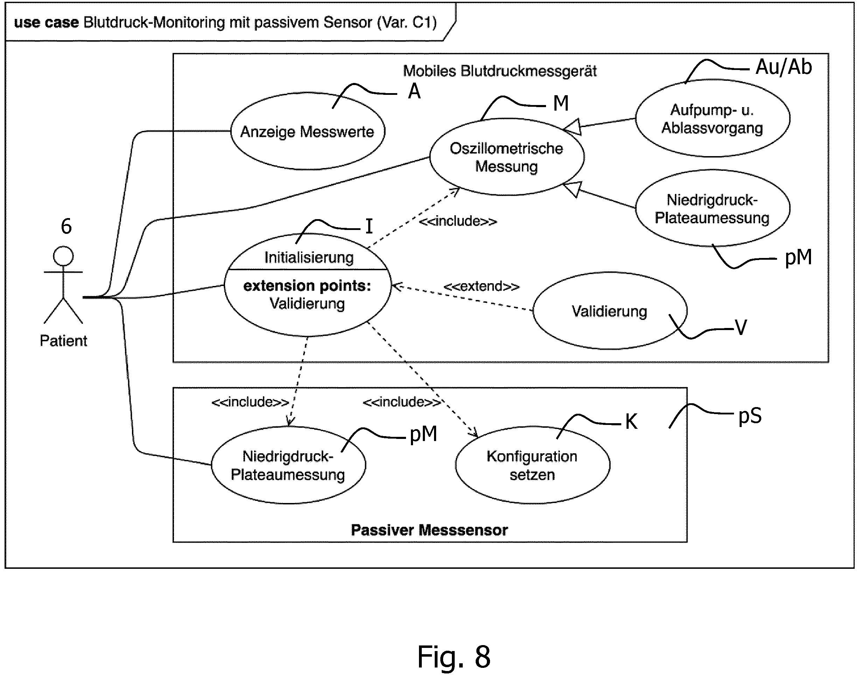

Fig. 8 zeigt bespielhaft einen weiteren beispielhaften Anwendungsfall C1. In diesem Ausführungsbeispiel wird die Blutdruckmessvorrichtung um einen passiven Messsensor pS ergänzt, der eine belastungsfreie Blutdruckmessung während des Messzeitraums ermöglichen soll. Passive Messsensoren in dem Kontext des hier beschriebenen Verfahrens zum Betreiben einer Blutdruckmessvorrichtung beschreiben Sensor-Messeinheiten, die keine oder eine vernachlässigbare oder in einem nicht regelbaren Ausmaß eine konstante Einwirkung auf den Patienten oder Anwender aufweisen. Z.B. sind Messeinheiten mit optischen Sensoren zur Erfassung eines PPG gemäß dieser Definition ein passiver Messsensor. Als passiver Sensor kann auch ein elektromagnetischer Sensor (z.B. Nahfeld-Radar) zur kontaktlosen Pulswellenmessung dienen. Zudem ist ein Pflaster oder Textil-Kompressionsstrumpf mit integrierten Dehnungssensoren ohne aktive Elemente zur Regelung der Kompressionsstufe ein passiver Sensor.

Anwendungsfall C2

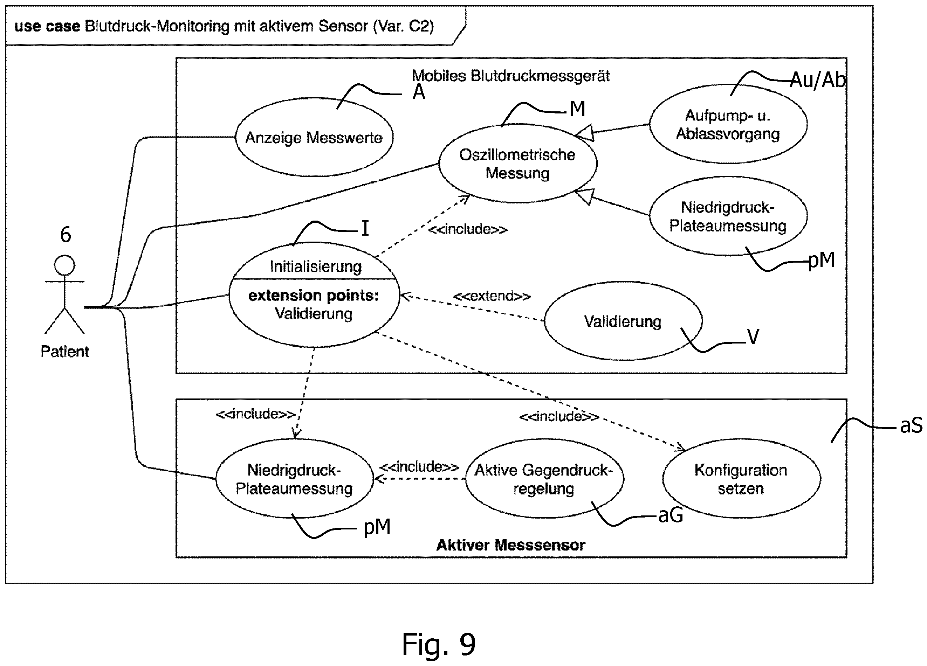

Bei dem in Fig. 9 gezeigten beispielhaften Anwendungsfall C2 wird die Blutdruckmessvorrichtung um einen aktiven Messsensor aS ergänzt. Der aktive Messsensor aS wird beispielsweise über eine oszillometrische Messung M analog zu den oben aufgeführten Ausführungsbeispielen in einer Initialisierung I initialisiert. Während der Niedrigdruckmessung pM kann durch eine aktive Gegendruckregelung aG der Anpressdruck der Messeinheit, sowie der von der Messeinheit auf den Patienten oder Anwender wirkende Gegendruck (und somit der in der peripheren Arterie vorherrschende Transmuraldruck) geregelt werden. Mögliche Elemente einer aktiven Gegendruckregelung aG sind die den elektronischen Regelkreis implementierende Elektronik, ein Aktor (z.B. ein Piezoelement oder eine elektromechanische Vorrichtung) und ein Druckmesssensor, der auch als Messsensor für die Blutdruckmessung verwendet werden kann.

Die aktive Gegendruckregelung aG kann beispielsweise einen oder mehrere vorgegebene Gegendrücke für einen tonometrischen Sensorkopf erzeugen. Ein weiteres Beispiel für eine aktive Gegendruckregelung ist die Einbettung von elektromechanischen Aktoren in einen Textilmessstrumpf, der in Abhängigkeit einer elektrischen, zeitveränderlichen oder konstanten Spannung seine Longitudinalauslenkung ändert und somit zu einer Änderung des Durchmessers des Textilmessstrumpfs führt. Die Änderung des Durchmessers des Textilmessstrumpfes bewirkt wiederum eine Änderung des auf die Arterie wirkenden Gegendrucks und somit des Transmuraldrucks.

Die aktive Gegendruckregelung befähigt den mit dem Blutdruckmessgerät verbundenen aktiven Messsensor Niedrigdruck-Plateaumessungen pM mit verschiedenen konstanten Gegendrücken durchzuführen. Beispielsweise kann eine Sequenz von Niedrigdruck-Plateaumessungen während eines Messvorgangs abgearbeitet werden, bei der eine Niedrigdruck-Plateaumessung mit einem konstanten Gegendruck von 50 mmHg auf den Patienten/Anwender wirkt, gefolgt von einer Niedrigdruck-Plateaumessung mit einem konstanten Gegendruck von 70 mmHg.

Anwendungsfall D

Eine mögliche Kombination aus den Anwendungsfällen B und C1 und/oder C2 stellt ein beispielhafter, hier nicht in den Figuren gezeigter Anwendungsfall D dar, bei dem das Blutdruck-Monitoring mit Niedrigdruck-Plateaumessungen im Rahmen eines stationären Aufenthaltes des Patienten in einer medizinischen Einrichtung, bspw. einem Krankenhaus, erfolgt. Patienten in stationärer und/oder intensivmedizinischer Behandlung stellen eine besonders vulnerable Patientengruppe dar, bei der einerseits eine kontinuierliche Überwachung möglichst aller relevanter, insbesondere kardiovaskulärer, Vitalparameter notwendig ist. Andererseits muss die Belastung für den Patienten und der Aufwand für das medizinische Personal möglichst geringgehalten werden.

Ein Ausführungsbeispiel gemäß Anwendungsfall D ist die Initialisierung einer Blutdruckmessvorrichtung mit einer Referenzmessung, z.B. einer invasiven Blutdruck- und Pulswellenmessung mittels arteriellen Katheters während eines routinemäßigen Diagnostik- oder Therapievorgangs. Mithilfe der aus der invasiven Referenzmessung bestimmten arteriellen Pulswellenaufzeichnung und der mit der nichtinvasiven Blutdruckmessvorrichtung aufgezeichneten Niedrigdruck-Plateaumessung (z.B. durch eine oszillometrische Messung, optische Messung, etc.) kann das personenspezifische Übertragungsverhalten von Arterie zum nichtinvasiven Messsensor im Rahmen der Initialisierung bestimmt werden. Danach ist eine belastungsarme Blutdruckmessung während des stationären Monitorings des Patienten mit einer einzelnen oder einer Kombination von Niedrigdruck-Plateaumessungen möglich.

Nachfolgend sollen beispielhafte Einzelheiten der Initialisierungsschritte und der dabei ablaufenden Signalverarbeitung sowie der Blutdruckmessungen und der Niedrigdruck-Blutdruckmessung und der dabei ablaufenden Signalverarbeitung näher erläutert werden. Zur Ausführung der nachfolgend beschriebenen Verfahrensschritte wird grundsätzlich auf die beispielhaft in Fig. 1 dargestellte und vorhergehend erläuterte Konfiguration zurückgegriffen. Sofern hiervon Abweichungen erforderlich sind, wird dies nachfolgend erwähnt.

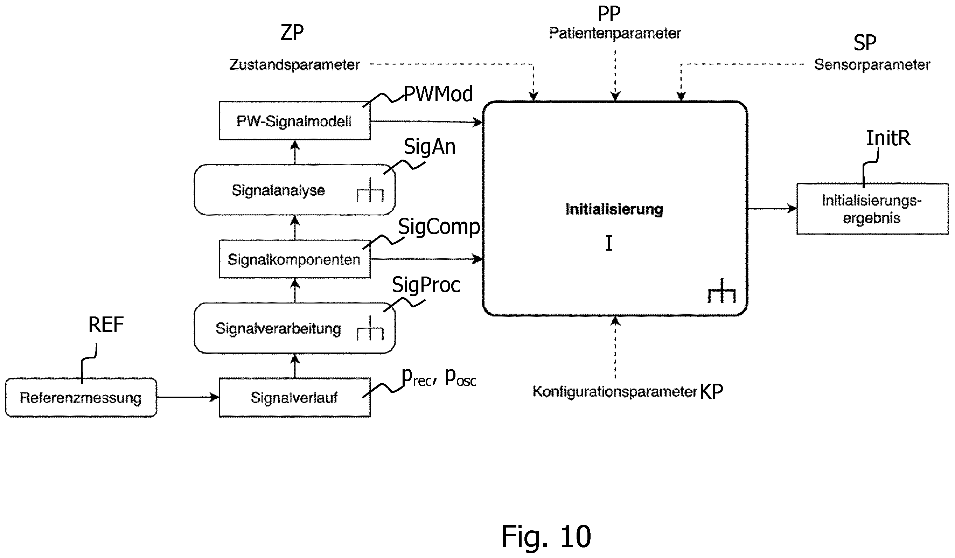

Wie im exemplarischen Messablauf in Fig. 5 dargestellt, erfolgt zu Beginn eines vorgegebenen Messzeitraums eine Initialisierung I des Verfahrens, gefolgt von einer Validierung V des Initialisierungsergebnisses. Die Initialisierung I des Verfahrens ist in Fig. 10 dargestellt. Diese ist Hauptbestandteil einer personenspezifischen Konfiguration einer Blutdruckmessvorrichtung gemäß Fig. 1 für die Durchführung einer Blutdruckmessung mithilfe von Niedrigdruck-Plateaumessungen.

In einer Referenzmessung REF erfolgt die Registrierung und Speicherung eines referentiellen zeitlichen Signalverlaufes $p_{rec}$ eines pulsierenden Signals, z.B. die direkte Erfassung der Pulswelle über eine invasive Blutdruckmessung oder die Erfassung des oszillierenden Signalanteils $p_{osc}$ einer oszillometrischen Messung mittels Blutdruckmanschette. Aus der Referenzmessung REF können, wenn nicht anderweitig vorhanden, die zum Zeitpunkt der Referenzmessung vorherrschenden Blutdruckwerte des Patienten oder Anwenders ermittelt und an die Initialisierung übergeben werden.

Mögliche Referenzsignale zur Initialisierung können insbesondere sein:
Messdaten ($p_{osc}$, $p_{cuff}$) aus einem vollständigen oder teilweisen Ablassvorgang Ab einer oszillometrischen Messung gemäß den vorhergehenden Anwendungsbeispielen.

Messdaten ($p_{osc}$, $p_{cuff}$) aus einem Aufpump- und/oder Ablassvorgang Au/Ab einer oszillometrischen Messung gemäß den vorhergehenden Anwendungsbeispielen.

Messdaten ($p_{osc}$, $p_{cuff}$) aus einem vollständigen/teilweisen Aufpumpvorgang Au einer oszillometrischen Messung gemäß den vorgehenden Anwendungsbeispielen.

Messdaten ($p_{osc}$, $p_{cuff}$) aus mindestens einem, vorzugsweise N Plateaumessungen pM bei N unterschiedlichen, konstanten Gegendrücken bei konstanter Patientenposition/-haltung gemäß den vorgehenden Anwendungsbeispielen.

Messdaten ($p_{osc}$, $p_{cuff}$) aus mindestens zwei, vorzugsweise N Plateaumessungen pM bei M≤N (möglicherweise unterschiedlichen), aber konstanten Gegendrücken gemäß den vorgehenden Anwendungsbeispielen, aber bei unterschiedlichen Patientenpositionen bzw. Patientenhaltungen (d.h. bei Durchführung verschiedener Manöver vor bzw. während der Blutdruckreferenzmessung, die zu einer Änderung des arteriellen Blutdrucks führen).

Messdaten ($p_{osc}$, $p_{cuff}$) bei einer Plateaumessung pM mit niedrigem, aber zeitvariablem Gegendruck.

Möglich ist auch die Verwendung von Messdaten ($p_{invasive}$) aus einer invasiven Pulswellen- und Blutdruckmessung mit Katheter in Verbindung mit einer gleichzeitigen oder zeitnahen oszillometrischen Messung in einer der oben genannten Ausführungen und Anwendungsfälle.

Nach der Referenzmessung REF erfolgt eine Signalverarbeitung SigProc, die aus dem Signalverlauf relevante Signalkomponenten SigComp für die nachfolgenden Vorverarbeitungsschritte und die Initialisierung I extrahiert.

Anhand der Signalkomponenten SigComp erfolgt eine ausführliche Signalanalyse SigAn zur Extraktion von Pulswellenmerkmalen und der Bildung eines PulswellenSignalmodells PWMod.

Notwendige Eingangsvariablen für den Initialisierungsprozess des Verfahrens sind die aus dem Signalverlauf extrahierten Signalkomponenten SigComp und das PW-Signalmodell PWMod. Optionale Schnittstellen stellen die Übergabe von Eingangsvariablen an die Initialisierung zur Verfügung, welche Zustandsparameter ZP der Blutdruckvorrichtung und des Verfahrens, Sensorparameter SP, Konfigurationsparameter KP und Patientenparameter PP beschreiben.

Zustandsparameter ZP beschreiben beispielsweise den Zeitpunkt der Initialisierung, vorherige Initialisierungen und deren Ergebnisse, den Zustand der Blutdruckmessvorrichtung oder den Zustand des Verfahrens.

Sensorparameter SP beschreiben beispielsweise die Art des Sensors (Drucksensor, mechanischer Sensor, elektromechanischer Sensor, optischer Sensor, elektromagnetischer Sensor), die Art der Messeinheit (z.B. passiv, aktiv), die Größe und Ausprägung des Sensors (z.B. Messposition, Manschettengröße, Wellenlängen, Gewicht, etc.).

Patientenparameter PP beschreiben beispielsweise das Alter, das Körpergewicht, die Körpergröße, das Geschlecht und den Zustand des Patienten oder Anwenders.

Konfigurationsparameter KP definieren beispielsweise die Beschreibungsart des Übertragungsverhaltens sowie die Domäne der Beschreibung/Berechnung des Übertragungsverhaltens (z.B. Zeitbereich, Frequenzbereich, Zeit-Frequenzbereich), den gewählten Modellierungsansatz, den zugrundeliegenden Modelltyp, den verwendeten Optimierungsalgorithmus und Definitions- und Wertebereiche.

Die Initialisierung I des Verfahrens stellt ein Initialisierungsergebnis InitR zur Verfügung, das u. a. die Koeffizienten des gesuchten, personenspezifischen Übertragungsverhaltens mit Güteparametern und Parametern zur Beschreibung des Initialisierungsprozesses (z.B. Dauer der Initialisierung, Iterationsschritte, Status der Initialisierung) enthält.

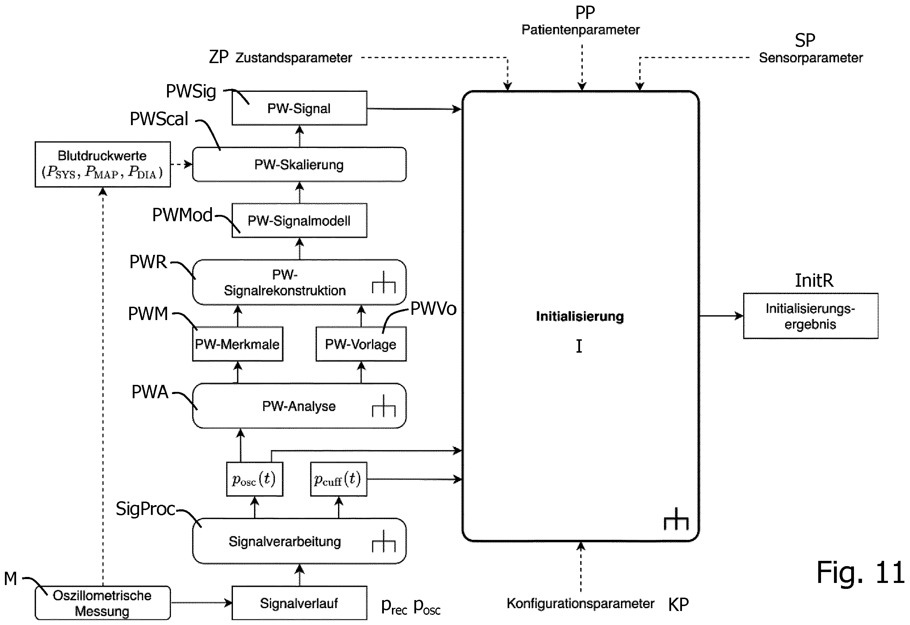

Fig. 11 zeigt beispielhafte Schnittstellen des Verfahrensschrittes der Initialisierung I mit Vorverarbeitungsschritten und Datenobjekten für ein Ausführungsbeispiel des vorgestellten Verfahrens bei Verwendung einer oszillometrischen Messung mit Auswertung des Ablassvorgangs. Der vorgegebene Spitzendruck kann dabei während der Referenzmessung über dem zu erwartenden systolischen Blutdruck liegen. Der vorgegebene Spitzendruck kann jedoch auch weit unterhalb des zu erwartenden systolischen Blutdrucks liegen (bei einer Niedrigdruck-Ablassvorgang mit teilweisem Aufpumpvorgang).

Wenn keine vollständige oszillometrische Messung durchgeführt wird, können die Referenzparameter aus anderen Messungen, insbesondere über einen Kathetersensor, oder aus der Historie vorhergehender Messungen bestimmt werden. Eine Erweiterung stellt die Bestimmung von Referenzparametern aus einer Klassifizierung von Pulswellen und einer Zuordnung von Pulswellenmerkmalen über eine empirische Gleichung einer zugeordneten Teilpopulation dar.

Aus der oszillometrischen Messung M wird der Signalverlauf vom Druckmesssensor ($p_{rec}$) ermittelt. Zudem werden (bspw. aus dem Ablassvorgang) die Blutdruckwerte für den systolischen ($P_{SYS}$), den diastolischen ($P_{DIA}$) sowie für den mittleren arteriellen ($P_{MAP}$) Blutdruck als Referenzwerte ermittelt.

In der Signalverarbeitung SigProc werden die Zeitverläufe der oszillierenden ($p_{osc}$) und nichtoszillierenden ($p_{cuff}$) Signalanteile bestimmt, die als Eingangsvariablen für die Initialisierung dienen.

Anhand des oszillierenden Signalanteils $p_{osc}$ wird anschließend eine Pulswellenanalyse PWA durchgeführt, aus der sich die Pulswellenmerkmale PWM und eine Pulswellenvorlage PWVo ergeben. In einer Pulswellen-Signalrekonstruktion PWR wird ein Pulswellen-Signalmodell PWMod aus der PW-Vorlage anhand der PW-Merkmale von $p_{osc}$ berechnet. Mithilfe der aus der Referenzmessung bekannten Blutdruckwerte wird das PW-Signalmodell in einer Pulswellenskalierung PWScal skaliert und ggf. transformiert, sodass das sich ergebende berechnete PW-Signal die arterielle Pulswelle möglichst genau approximiert.

Es erfolgt eine Übergabe von $p_{osc}$, $p_{cuff}$, PW-Signal PWSig, Zustandsparameter ZP, Patientenparameter PP, Sensorparameter SP und Konfigurationsparameter KP an den Prozess der Initialisierung I und die Bestimmung des Initialisierungsergebnisses durch Lösung eines Optimierungsproblems, welches eine berechnete Antwort p'$_{osc}$ möglichst genau auf $p_{osc}$ abbildet, d.h. die berechneten Koeffizienten zur Nachbildung des Übertragungsverhaltens bilden das PW-Signal möglichst genau auf $p_{osc}$ ab.

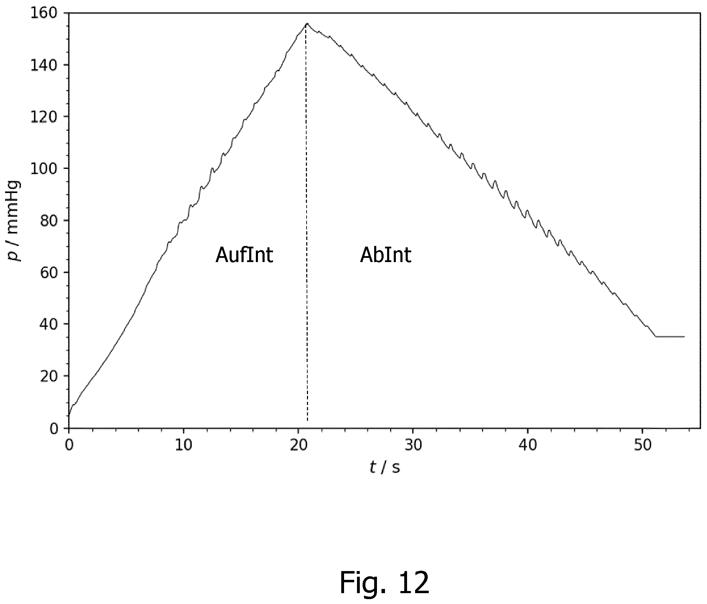

Ein beispielhaftes oszillometrisches Messsignal ist in Fig. 12 dargestellt. Im Ausführungsbeispiel nach Fig. 11 erfolgt eine Vorverarbeitung des oszillometrischen Messsignals mit folgenden Schritten: Es erfolgt zunächst gegebenenfalls eine Abtastratenerhöhung mithilfe einer Interpolation, vorzugsweise einer kubischen Interpolation.

Danach erfolgt eine Trennung in oszillierende und nichtoszillierende Signalkomponenten ($p_{osc}$, $p_{cuff}$). In dem Beispiel aus Fig. 12 macht sich die oszillierende Signalkomponente als Zackenstruktur bemerkbar, die dem dreiecksförmigen glatten Verlauf des Drucks bei dem Aufpump- und Ablassvorgang überlagert ist.

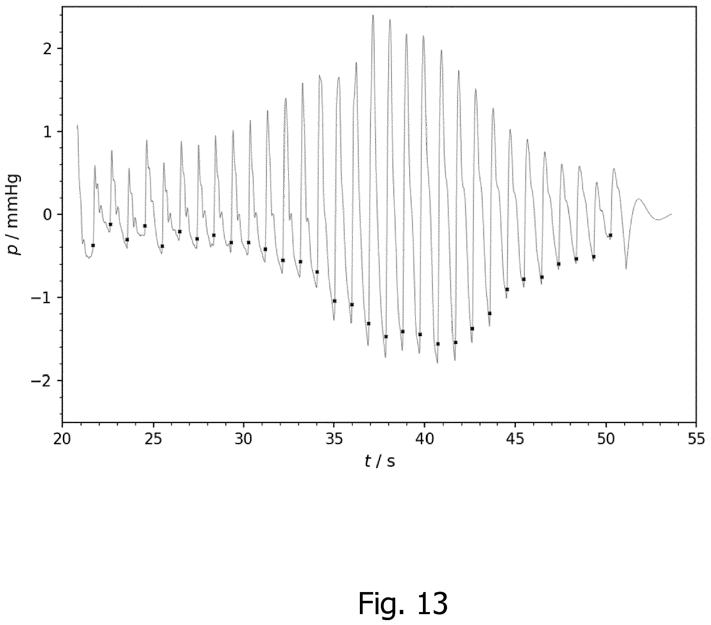

Es erfolgt dann eine Intervallbestimmung der nichtoszillierenden Signalkomponente, und zwar in ein Aufpumpintervall AufInt, ein Ablassintervall AbInt, und ggf. in ein Intervall mit konstantem Gegendruck. Fig. 13 zeigt beispielhaft den oszillierenden Signalanteil $p_{osc}$,

der aus dem Ablassintervall absepariert worden ist.

In der Signalverarbeitung SigProc erfolgt nach den oben genannten Schritten der Vorverarbeitung eine Artefakterkennung in den nichtoszillierenden und oszillierenden Signalanteilen. Dabei werden eine Identifikation und Klassifikation von Artefakten im Messsignal sowie deren Auftrittszeitpunkte bzw. -intervalle durchgeführt. Dieser Teilschritt dient als Grundlage zum Ausschluss und der Verminderung der Artefakte durch geeignete Signalverarbeitungsmethoden (z.B. Filterung, Signalzerlegung mit multivariater Statistik, Signalmanipulation, etc.). Die Artefakterkennung ermöglicht zudem die Definition von artefaktfreien Zeitintervallen, sogenannten Regions of Interest (ROIs), die eine fehlerbereinigte Initialisierung und modellbasierte Blutdruckbestimmung ermöglichen.

Aus dem artfefaktbereinigten Referenzsignal kann optional ein für die Initialisierung und/oder modellbasierte Blutdruckbestimmung ein geeigneter Signalabschnitt extrahiert werden. Dieser Signalabschnitt kann beispielsweise 1 bis N aufeinanderfolgende Pulswellen enthalten.

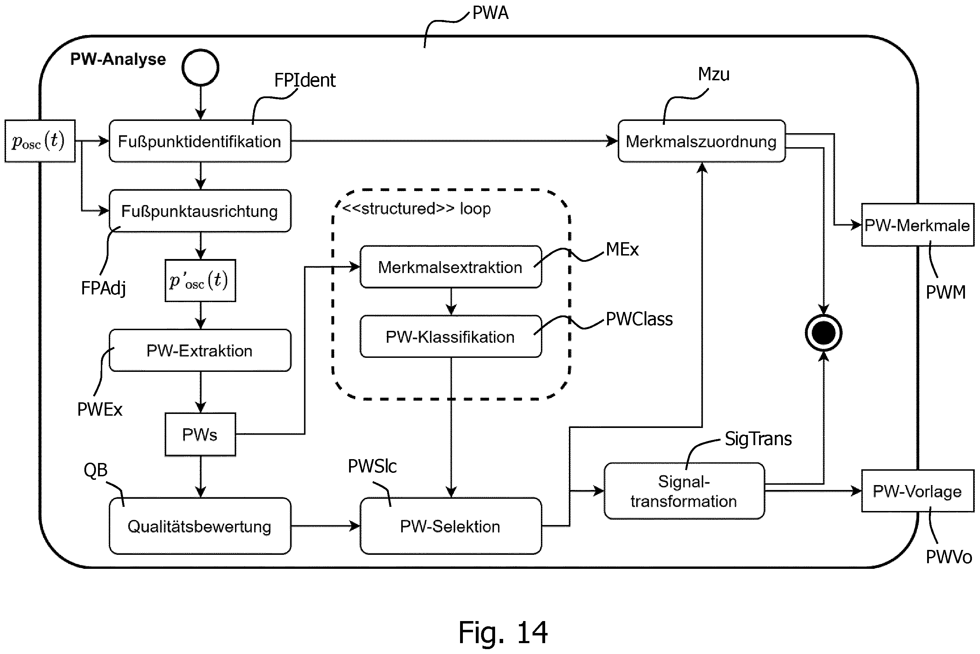

In der beispielhaft in Fig. 14 gezeigten Pulswellenanalyse PWA werden aus dem oszillierenden Signalanteil der Manschettenmessung Pulswellenmerkmale PWM und eine Pulswellenvorlage PWVo bestimmt.

Ziel der PW-Vorlage ist die detailgetreue Abbildung einer gemittelten arteriellen Pulswellenform in der peripheren Arterie (z.B. in der A. brachialis). Dabei werden in der PW-Analyse aus dem Eingangssignal für die Initialisierung eine Menge von Pulswellen (mindestens jedoch eine Pulswelle) extrahiert und daraus nach einer Klassifikation der Pulswellen durch eine geeignete Signaltransformation eine Pulswellenvorlage erstellt. Dabei werden die nachfolgend aufgeführten Schritte durchgeführt.

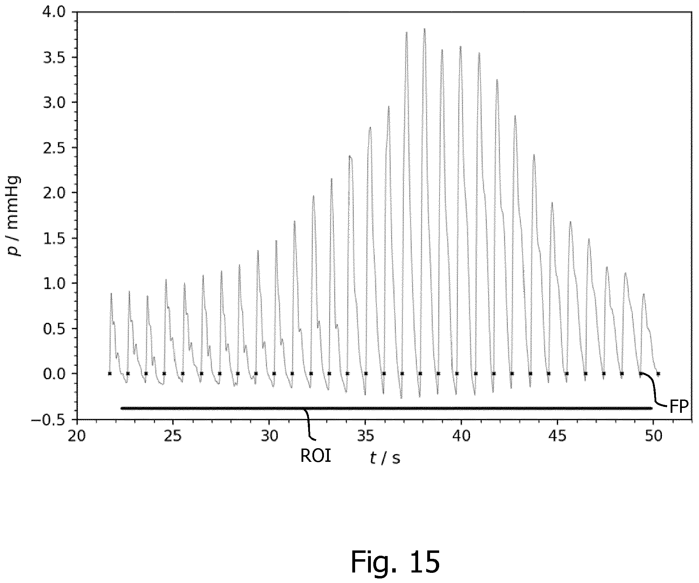

Es erfolgt eine Segmentierung des oszillierenden Signalanteils in einzelne Pulswellen und Bestimmung der zugehörigen Pulswellen-Fußpunkte FP. Anschließend wird eine Ausrichtung des oszillierenden Signalanteils an den Fußpunkten durch Interpolation durchgeführt. Ein Beispiel hierfür ist in Fig. 15 gezeigt. An den Pulswellen-Fußpunkten FP ist hier der oszillierende Signalanteil $p_{osc}$ für das Beispiel aus Fig. 13 ausgerichtet. Die schwarzen Basislinien stellen beispielhafte Markierung von artefaktfreien Auswertebereichen oder Regions of Interest (ROIs) dar. Es erfolgt hierzu zunächst eine Artefakterkennung unter Berücksichtigung des nichtoszillierenden Signalanteils.

Es erfolgt eine Zerlegung des ausgerichteten, oszillierenden Signalanteils in Daten über Einzelperioden zum Identifizieren von einzelnen Pulswellen.

Nachfolgend wird eine Klassifizierung und Qualitätsbewertung der einzelnen Pulswellen anhand der Pulswellenmorphologie und der individuellen Pulswellenmerkmale durchgeführt. Anhand des Klassifizierungsergebnisses wird eine Teilmenge von extrahierten Pulswellen gebildet.

Die Klassifizierung kann anhand der Pulswellenmorphologie, der Pulswellenmerkmale oder vorgegebener Messparameter erfolgen. Dabei ist eine geeignete Kombination von Klassifizierungsregeln möglich und vorteilhaft. Beispielsweise werden Pulswellen nur aus dem suprasystolischen Bereich $P_{cuff} > P_{SYS}$ berücksichtigt, die eine gleiche oder ähnliche Pulswellenform aufweisen.

Die Pulswellenanalyse gemäß Fig. 14 beginnt mit einem Einlesen des oszillierenden Signalsanteil $p_{osc}$ (t). Hieran wird eine Fußpunktidentifikation FPIdent ausgeführt. Den über den so ermittelten Fußpunkten erfassten einzelnen Pulswellen wird in einer Merkmalszuordnung MZu eine Klassifikation zugewiesen.

Die identifizierten Fußpunkte werden über eine Fußpunktausrichtung FPAdj ausgerichtet und im Wesentlichen auf einen bestimmten Signaloffset normiert. Dies bedeutet vor allem die Ausrichtung auf eine vorgegebene Nulllinie.

Es entsteht dadurch ein ausgerichteter oszillierender Signalanteil $p'_{osc}(t)$. An diesem ausgerichteten oszillierenden Signalanteil erfolgt die Pulswellenextraktion PWEx.

Die extrahierten Pulswellen PW werden in einer Qualitätsbewertung QB qualitativ bewertet, dies betrifft insbesondere das Ausselektieren von artefaktbehafteten Signalverläufen und das Auswählen von Pulswellen in bestimmten Druckbereichen während des Ablassvorgangs oder bestimmter Pulswellenformen.

In Verbindung erfolgt damit das Ausführen einer Merkmalsextraktion MEx an den einzelnen Pulswellen und eine Pulswellenklassifikation PWClas. Die Schritte QB, MEx und PWClass ergeben eine Pulswellen-Selektion PWSlc, die für den Schritt der Merkmalszuordnung MZu die Grundlage bildet.

Ausgehend von der Pulswellenselektion PWSlc erfolgt eine Signaltransformation SigTrans zum Gewinnen einer Pulswellenvorlage PWVo.

Die Signaltransformation SigTrans berücksichtigt eine variierende Hämodynamik (z.B. durch eine Änderung der Herzfrequenz) durch geeignete Operationen (z.B. dynamische Zeitentzerrung [Dynamik Time Warping], nichtlineare/lineare Skalierung, Interpolation, digitale Filterung). Zudem erfolgt eine Amplitudennormierung der einzelnen Pulswellen. Die extrahierten Pulswellen können mit unterschiedlicher Wichtung in die Signaltransformation SigTrans eingehen.

Ein mögliches Beispiel für eine einfache Signaltransformation ist die gewichtete Mittelung von mit nichtlinearen Skalierungsoperationen zueinander ausgerichteten Pulswellen. Vorstellbare alternative Signaltransformationen können durch Funktionsapproximation mit neuronalen Netzen (oder weiteren Methoden des maschinellen Lernens), FIR-Filterbänken, Wavelet-Zerlegungen und/oder Signalzerlegung mit Verfahren multivariater Statistik (SVD, PCA, ICA) erfolgen.

Es wird eine Identifikation von geeigneten Pulswellenmerkmalen für die PW-Vorlage durchgeführt, um im nächsten Schritt eine PW-Signalrekonstruktion durchführen zu können. Weiterhin können die ermittelten Pulswellenmerkmale zur Klassifikation des Patienten/Anwenders und/oder seines Zustands verwendet werden, um auf der Grundlage des Klassifizierungsergebnisses in nachfolgenden Schritten personenspezifische Algorithmen und/oder Parameter auszuwählen und/oder zu generieren.

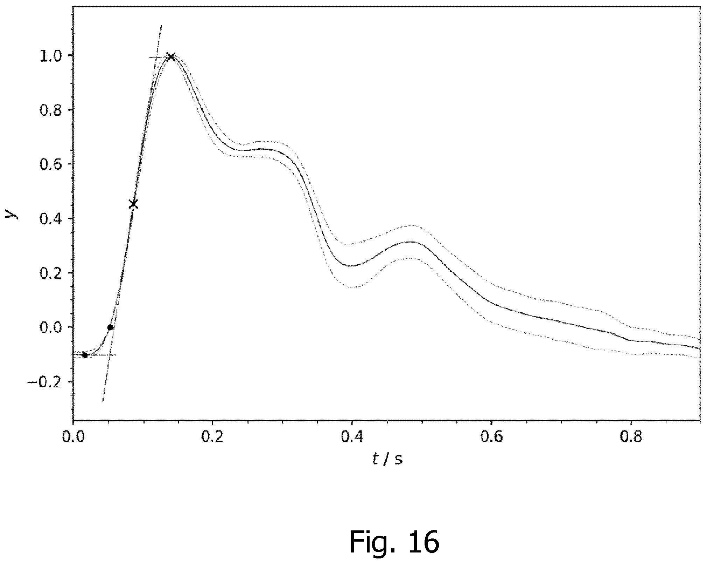

Fig. 16 zeigt als durchgehende Line eine Pulswellenvorlage (schwarz) mit beispielhaften Pulswellenmerkmalen (kreuzförmige und punktförmige Markierungen), die aus einem Tangentenschnittpunkt-Verfahren (Strichlinien) hervorgehen. Die Punktlinien stellen Unsicherheitsbereiche abgeleitet aus der zur PW-Vorlage verwendeten einzelnen Pulswellen, bspw. aus einer (gewichteten) Standardabweichung dar.

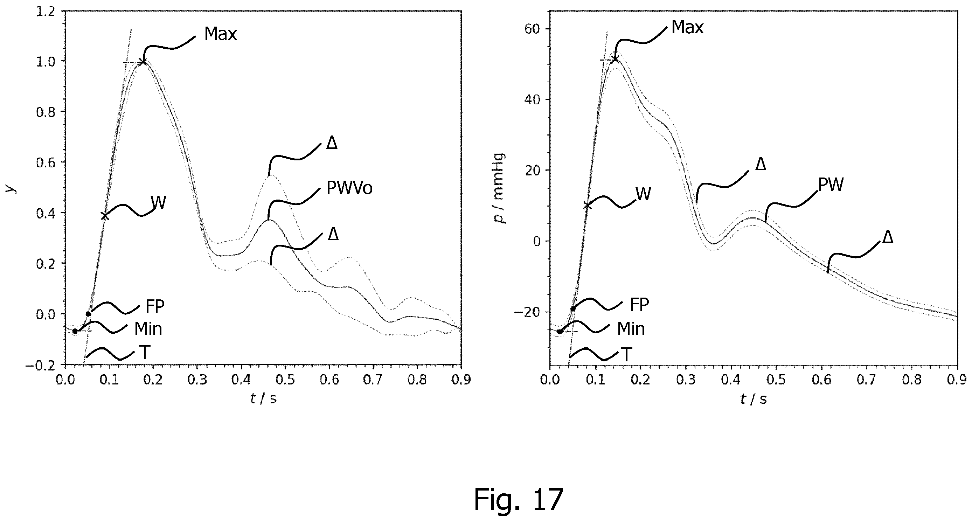

Fig. 17 zeigt links ein Beispiel für eine aus dem suprasystolischen Bereich des oszillometrischen Signalanteils mit einer Manschettenmessung bestimmten Pulswellenvorlage PWVo. Rechts ist eine mittelwertbereinigte, gemittelte Pulswelle PW einer invasiven Blutdruckmessung mittels Katheter aus der A. brachialis im selben Patienten, gegenüberliegender Arm dargestellt. Das Beispiel zeigt einen Fall, bei dem die PW-Merkmale und PW-Form zwischen PW-Vorlage und invasiver Pulswelle eine gute Übereinstimmung aufweisen.

Dargestellt sind entsprechende Confidence-Bereiche $\Delta$. Die übereinstimmenden Parameter beider Verläufe zeigen sich insbesondere in der Übereinstimmung der Formen innerhalb des vorliegenden Zeitbereiches sowie der zeitlichen Position einzelner Kurvenpunkte, beispielsweise der Maxima Max, Wendepunkte W, Fußpunkte FP, Minima Min, sowie Tangentenverläufe T.

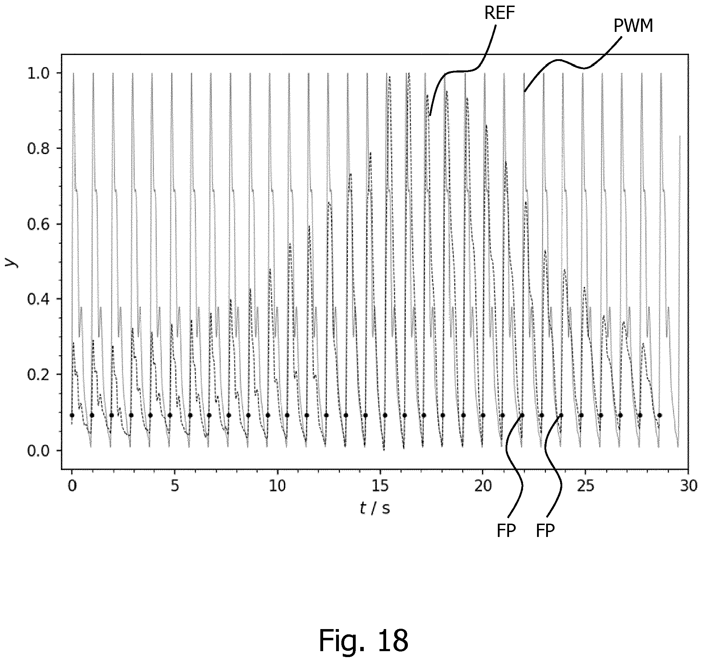

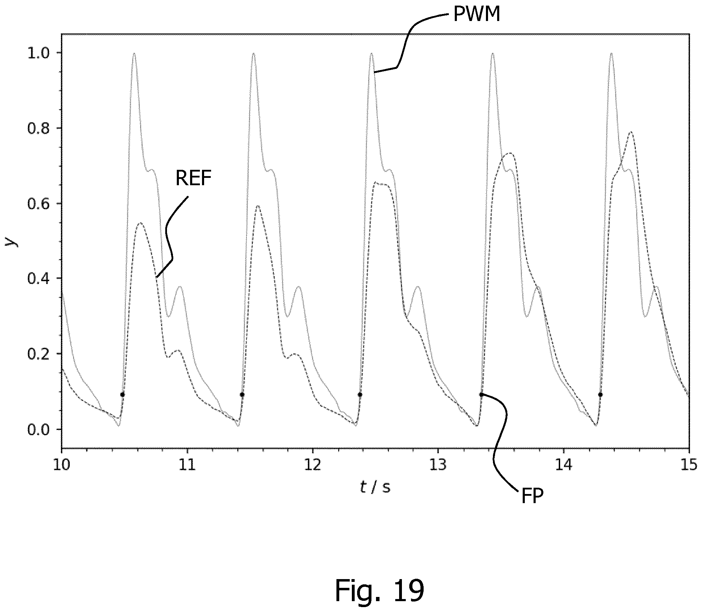

Anhand von aus dem Referenzsignal bestimmten Pulswellenmerkmalen (z.B. Zeitpunkt der mit Tangentenschnittpunktverfahren ermittelten Pulswellen-Fußpunkte) erfolgt eine Rekonstruktion eines zum oszillierenden Signalanteils des Referenzsignals zeitsynchronen Pulswellensignalmodell PWM, gemäß den Figuren 18 und 19.

Dazu werden aus dem Zeitverlauf des Referenzsignals (insbesondere die Zeitpunkte geeigneter Pulswellenmerkmale, bspw. der Pulswellenminima, Fußpunkte FP, Pulswellenmaxima) Skalierungsfaktoren und Pulswellenabstände bestimmt. Anhand dieser wird aus der Pulswellenvorlage ein zum Referenzsignal zeitsynchrones Pulswellenmodell durch Interpolation (und ggf. Extrapolation) erstellt.

Durch die Wahl der PW-Vorlage erfolgt die Bildung eines personenspezifischen und klassenspezifischen PW-Signalmodells, welches sowohl konkrete Eigenschaften aus dem vom Patienten bzw. Anwender erfassten oszillierenden Signalanteil berücksichtigt, als auch Parameter aus einer Datenbank infolge des Klassifizierungsergebnisses der Pulswellenmerkmale und Pulswellenform in Kombination mit personenspezifischen und/oder sensorspezifischen Parametern (z.B.

Geschlecht, Alter des Patienten/Anwenders).

Es erfolgt anschließend eine Verkettung von skalierten Pulswellenvorlagen in Verbindung mit einer kubischen Interpolation zur Vermeidung von Signalsprüngen und anderer Unstetigkeitsstellen. Dabei wird die zeitvariante Periodizität der erfassten Pulswelle berücksichtigt, z.B. infolge einer zeitlichen Veränderung der Herzrate, und durch Skalierung der PW-Vorlage entlang der Zeitachse in der Rekonstruktion des PW-Signalmodells aufgenommen.

Fig. 18 zeigt ein aus verketteten und skalierten PW-Vorlagen rekonstruiertes PW-Signalmodell PWM, welches die zeitvariante Hämodynamik des Referenzsignals (schwarze Strichlinie) an detektierten Fußpunkten FP berücksichtigt. Fig. 19 zeigt einen Ausschnitt aus Fig. 18.

Aus dem rekonstruierten PW-Signalmodell wird anhand der aus der Referenzmessung abgeleiteten oder den vom medizinischen Personal vorgegebenen Blutdruckwerten ein approximiertes Pulswellen-Signal axPW bestimmt, dass die arterielle Pulswelle möglichst genau approximiert. Dies kann durch eine geeignete Signaltransformation, bspw. einer einfachen Skalierung, erfolgen.

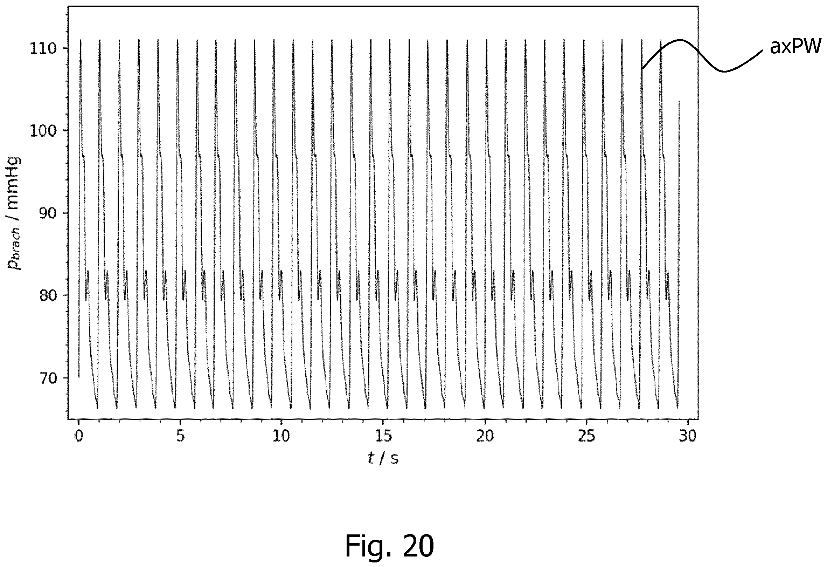

Fig. 20 zeigt beispielhaft ein solches dem PW-Signalmodell durch Skalierung mit den Blutdruckwerten $P_{SYS}$ und $P_{DIA}$ berechnetes approximiertes Pulswellensignal axPW als Eingangsvariable für die Initialisierung.

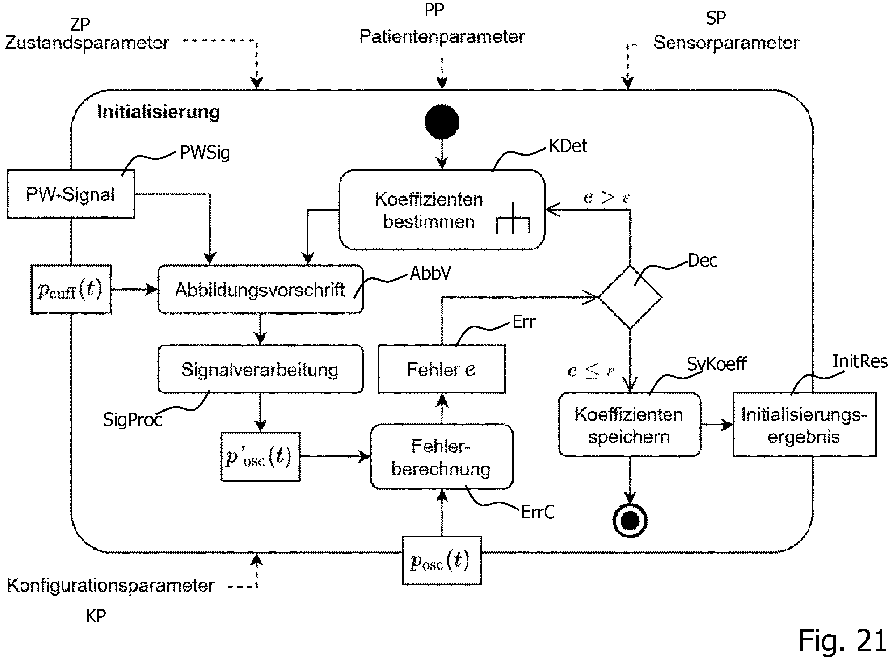

Fig. 21 zeigt einen beispielhaften Ablauf der iterativen Initialisierung I zur Bestimmung von personenspezifischen Koeffizienten für die das Übertragungsverhalten nachbildende mathematische Abbildungsvorschrift, bspw. durch Ausgleichsrechnung zwischen dem berechneten ($p'_{osc}$) und dem gemessenen ($p_{osc}$) oszillierenden Signalanteil.

Ziel der Initialisierung ist die Koeffizientenbestimmung KDet der personen- und sensorspezifischen Übertragungsfunktion, die das Übertragungsverhalten von peripherer Arterie (z.B. A. brachialis) zum Messsensor (z.B. Drucksensor in Blutdruckmanschette) beschreibt wie in Fig. 3 vorhergehend erwähnt. Die Übertragungsfunktion wird durch eine mathematische Abbildungsvorschrift beschrieben, deren Struktur bspw. durch ein neuronales Netz, eine Filterbank oder ein Differentialgleichungssystem vorgegeben werden kann.

Fig. 21 zeigt den iterativen Ablauf der Initialisierung I für eine oszillometrische Referenzmessung, bei der die Koeffizienten der Abbildungsvorschrift solange angepasst werden, bis die Abbildungsvorschrift das PW-Signal PWSig unter Berücksichtigung des Manschettendrucks $p_{cuff}(t)$ in eine berechnete Antwort transformiert, aus der ein oszillierender Signalanteil $p'_{osc}(t)$ extrahiert werden kann, der eine möglichst hohe Übereinstimmung mit dem aus der Referenzmessung extrahierten oszillierenden Signalanteil $p_{osc}(t)$ aufweist. Der iterative Prozess wird abgebrochen, wenn die Abweichung bzw. der Fehler e zwischen dem berechneten und dem gemessenen oszillierenden Signalanteil einen vorgegebenen Minimalfehler $\varepsilon$ erreicht oder unterschreitet.

Die Initialisierung stellt ein Optimierungsproblem dar, welches eine vorgegebene Kostenfunktion (Abweichung zwischen oszillierenden Signalkomponenten) minimieren soll. Dieses Optimierungsproblem kann z.B. mithilfe typischer globaler multivariater Optimierer gelöst werden.

Als Abbildungsvorschrift ist insbesondere ein modellbasierter Ansatz unter Verwendung eines Differentialgleichungssystems erster Ordnung zur Definition der Struktur und des Verhaltens geeignet.

Der hier in Fig. 21 dargestellte Prozess der Initialisierung I wird beispielsweise wie folgt ausgeführt: Zunächst werden eine Reihe von Zustandsparametern ZP, Patientenparametern PP des Patienten oder Anwenders und Sensorparametern SP als Randbedingungen für den Initialisierungsprozess vorgegeben. Zusätzliche Konfigurationsparameter KP betreffen die Art und Weise der Initialisierung, beispielsweise die Vorgabe von Fehlerschranken, bestimmte iterative Näherungsverfahren und dergleichen Parameter mehr.

Das Pulswellensignal PWSig sowie der anliegende zeitabhängige Manschettendruck $p_{cuff}(t)$ werden mit anfänglich gegebenen Koeffizienten der Übertragungsfunktion über eine Abbildungsvorschrift AbbV im Rahmen einer Signalverarbeitung SigProc in einen modellierten oszillierenden Signalanteil $p'_{osc}(t)$ überführt.

Der modellierte oszillierende Signalanteil $p'_{osc}(t)$ wird mit dem aus der Referenzmessung bestimmten oszillierenden Signalanteil $p_{osc}(t)$ verglichen. Es erfolgt dabei eine Fehlerberechnung ErrC und die Ausgabe Err eines Fehlers e.

Der Fehler e wird nachfolgend in einem Vergleichsschritt Dec mit einer vorgegebenen Fehlerschranke $\varepsilon$ verglichen. Ist der Fehler e größer als die Fehlerschranke $\varepsilon$, so werden in einem erneuten Iterationsschritt und einem erneuten Ausführen der Koeffizientenbestimmung KDet die Koeffizienten der Übertragungsfunktion neu bestimmt.

Ist der Fehler e kleiner als die Fehlerschranke $\varepsilon$, so werden die nun vorliegenden Koeffizienten der Übertragungsfunktion in einem Schritt SvKoeff gespeichert und als Initialisierungsergebnis InitRes ausgegeben.

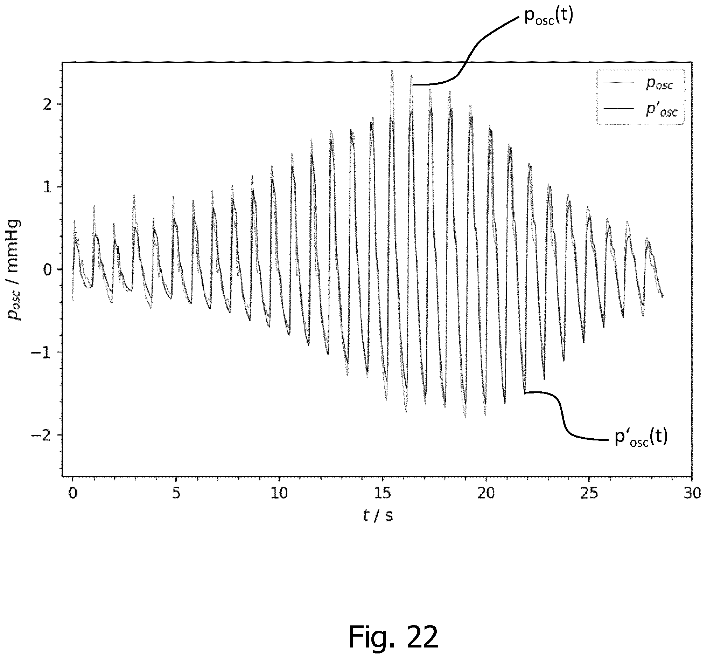

Fig. 22 zeigt ein beispielhaftes Ergebnis nach erfolgreicher Initialisierung. Der berechnete oszillierende Signalanteil $p'_{osc}(t)$ und der aus der Referenzmessung extrahierte oszillierende Signalanteil $p_{osc}(t)$ für einen vollständigen Ablassvorgang einer oszillometrischen Messung mit einer Blutdruckmanschette stimmen in ihren Signalverläufen optimal überein.

Nach Abschluss der Initialisierung werden die Koeffizienten der Übertragungsfunktion und deren statistische Unsicherheiten sowie Werte/Verlaufsparameter zur Charakterisierung des Initialisierungsprozesses (z.B. Wert der Kostenfunktion, benötigte Iterationen, etc.) im Initialisierungsergebnis gespeichert.

Eine geeignete Methode zur Bestimmung der Abbildungsvorschrift zwischen PW-Signal und vom Sensor erfassten Messsignal (bestehend aus den oszillierenden und den nichtoszillierenden Signalanteilen) ist die Modellierung des Messprozesses mithilfe eines geeigneten Differentialgleichungssystems.

Für das Ausführungsbeispiel einer manschettenbasierten oszillometrischen Blutdruckmessvorrichtung müssen somit Differentialgleichungen für die Druck- und Volumenänderung in der peripheren Arterie und für Druck- und Volumenänderungen in der Blutdruckmanschette mathematisch beschrieben werden. Die Aufstellung vereinfachter Modelle und zugehöriger Gleichungssysteme zur Beschreibung der Übertragung des arteriellen Volumen-Druck-Signals (d.h. der Pulswelle) zum Messsensor der Blutdruckmanschette ist in der Literatur bekannt.

Die Herausforderung besteht jedoch in der Anpassung der Modellbeschreibung und der Einbindung in das beschriebene Verfahren in einer Art und Weise, sodass eine eindeutige Beziehung zwischen PW-Merkmalen in der arteriellen Pulswelle und den PW-Merkmalen des pulsartigen gemessenen Signals bei konstantem, niedrigem Gegendruck der Niedrigdruck-Plateaumessungen in der Initialisierung bestimmt wird.

Ein wesentlicher charakteristischer Lösungsansatz ist die Verwendung einer geeigneten PW-Vorlage zur Rekonstruktion eines arteriellen PW-Signalmodells, welches die Morphologie der personenspezifischen arteriellen Pulswelle abbildet. Durch die Verwendung der Pulswellenvorlage mithilfe der in der Initialisierung vorliegenden Referenzblutdruckwerte wird die Problemstellung zu einem Vorwärtsproblem vereinfacht, bei dem aus einem bekannten Quellsignal ein Antwortsignal bestimmt wird. Da Quellsignale (rekonstruiertes arterielles PW-Signal und nichtoszillierender Manschettengegendruck) und Antwortsignal vorliegen, können die Koeffizienten des das Übertragungsverhalten nachbildenden Differentialgleichungssystem im Rahmen der Initialisierung iterativ berechnet werden. Die Bestimmung der Modellparameter erfolgt somit durch Lösen eines multivariaten Optimierungsproblems.

Mögliche Koeffizienten werden im modellbasierten Ansatz auch als Modellparameter bezeichnet, die in einem Spaltenvektor $x = [x_1, x_2, ..., x_n]^T$ angeordnet sind. Die Modellparameter beschreiben sowohl den zeitinvarianten Teil der manschettendruckabhängigen Übertragungsfunktion $H_{k,filt}$ als auch den zeitvarianten Teil $H_{k,hemo}$, der vom hämodynamischen Zustand des Patienten oder Anwenders abhängig ist.

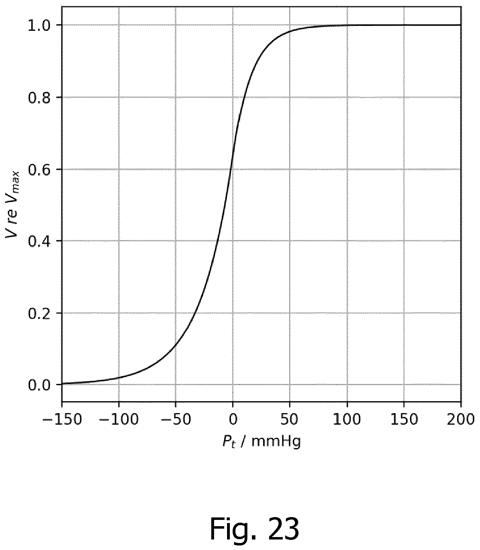

Beispiele für Modellparameter von $H_{k,filt}$ sind Parameter, die die dynamischen Filtereigenschaften des Gewebes (und ggf. des Sensors) und die allgemeine patientenspezifische Transmuraldruck-Volumen-Beziehung des Patienten/Anwenders charakterisieren. Eine derartige Beziehung ist beispielhaft in Fig. 23 dargestellt.

Modellparameter, die den zeitvarianten Teil $H_{k,hemo}$ charakterisieren, sind Parameter, die zeitvariable Effekte des Übertragungsverhaltens berücksichtigen, z.B. ein geänderter Durchmesser der peripheren Arterie durch Kontraktion der arteriellen Muskulatur, Einflüsse pharmakologischer Behandlung, etc. Änderungen in der zentralen Hämodynamik und Auswirkungen auf das periphere Pulswellensignal werden durch Transformationen der Pulswellenvorlage berücksichtigt.

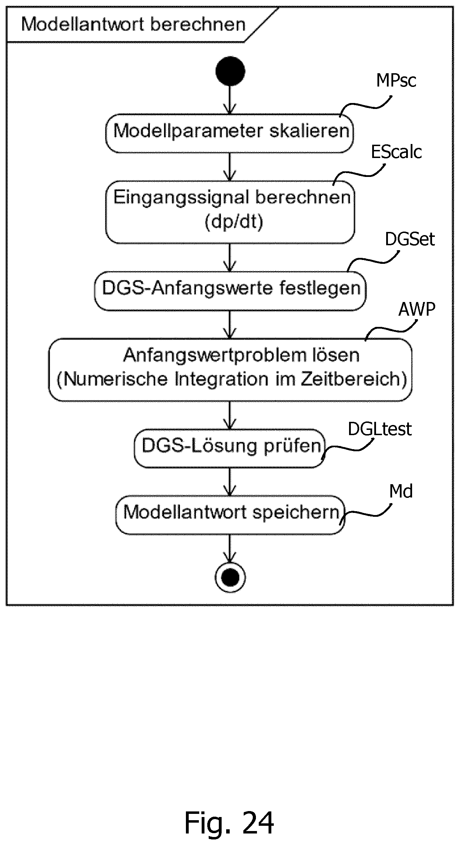

Nach Bestimmung der Modellparameter kann anhand des aus der PW-Vorlage rekonstruierten PW-Signals durch Lösen eines Anfangswertproblems für das gegebene Differentialgleichungssystem das vom Sensor erfasste Messsignal berechnet werden. Dies ist beispielhaft in Fig. 24 dargestellt.

Fig. 24 zeigt beispielhafte Verfahrensschritte zur Berechnung einer Modellantwort als einem wesentlichen Bestandteil der Initialisierung einerseits und somit auch der Durchführung der modellbasierten Blutdruckbestimmung andererseits. Der Messvorgang der Blutdruckmessung, bspw. einer oszillometrischen Messung, wird durch ein Differentialgleichungssystem (DGS) n-ter Ordnung abgebildet. Die Bestimmung der Modellantwort stellt ein Anfangswertproblem dar und kann durch numerische Integration des DGS gelöst werden. Dabei wird die Modellantwort, welche das gemessene Signal, z.B. das oszillometrische Messsignal, nachbildet, durch den Verlauf des arteriellen Pulswellensignals bestimmt, welcher als Ableitung als Eingangssignal an den Integrator übergeben wird.

Wenn das mit der Blutdruckmanschette erfasste Messsignal unter Änderung des nichtoszillierenden Manschettendrucks erfasst wurde, so muss als Eingangsparameter für das Differentialgleichungssystem die zeitabhängige Manschettendruckänderung vorliegen. Diese entspricht der numerisch berechneten Ableitung des nichtoszillierenden Manschettendrucksignals $p_{cuff}(t)$. Die berechnete Manschettendruckänderung kann durch Interpolation, Extrapolation und Ausgleichsrechnung modifiziert werden. Bspw. kann eine Extrapolation eine Berechnung der Modellantwort auch in Zeit- und Druckbereichen ermöglichen, zu denen keine Messdaten vorliegen. Die Extrapolation kann durch verschiedene mathematische Modelle berechnet werden. Eine mögliche Erweiterung ist die Bestimmung einer modellbasierten, geglätteten Manschettendruckänderung durch Ausgleichsrechnung der aus den Messdaten bestimmten Manschettendruckänderung mit einem geeigneten mathematischen Modell (z.B. lineares Modell, Exponentialmodell, Polynom).

Das Berechnen der Modellantwort erfolgt somit im Wesentlichen mit den Schritten des Skalierens von Modellparametern MPsc, daraufhin das Berechnen eines Eingangssignals EScalc, dem Festlegen der Anfangswerte des Differentialgleichungssystems DGSet, dem daraufhin folgenden Lösen des Anfangswertproblems AWP durch ein numerisches Integrieren im Zeitbereich, einem Prüfen der ermittelten Lösung DGLtest und dem Speichern der Modellantwort Md.

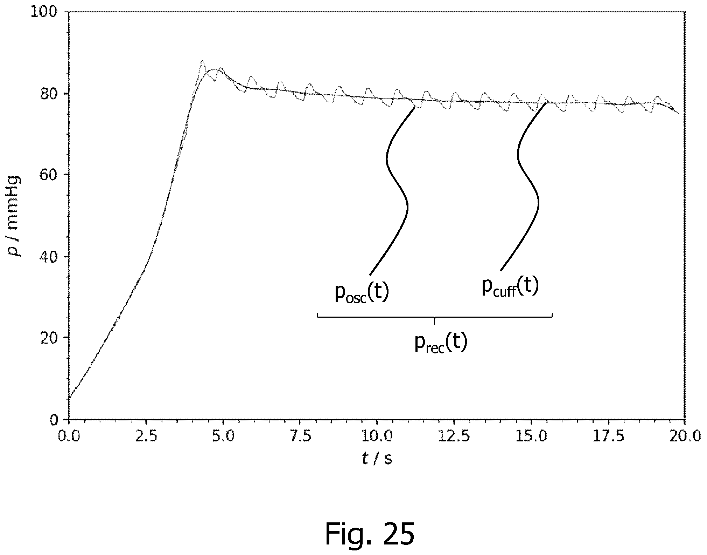

Erfindungsgemäß wird nach der Initialisierung und der Bestimmung der Koeffizienten der Übertragungsfunktion mit einer oszillometrischen Niedrigdruck-Plateaumessung gemäß Fig. 25 und der zum konstanten Plateau-Druck zugehörigen Übertragungsfunktion $H_k(j\omega)$ aus dem oszillierenden Signalanteil der Niedrigdruck-Plateaumessung die arterielle Pulswelle berechnet.

Fig. 25 zeigt einen beispielhaften Zeitverlauf einer aufgezeichneten oszillometrischen Niedrigdruck-Plateaumessung $p_{rec}(t)$ mit Blutdruckmanschette. Dieser stellt sich als eine Überlagerung aus einem oszillierenden Signalanteil $p_{osc}(t)$ und einem nicht oszillierenden Signalanteil $p_{cuff}(t)$ dar $P_{rec}(t) = p_{cuff}(t) + p_{osc}(t)$.

Die Niedrigdruck-Plateaumessung wird beispielsweise so ausgeführt, dass mittels einer Manschette ein Druck bis zu einem subsystolen Druck erhöht und dort einige Sekunden konstant auf einem Plateauwert gehalten wird. Der innerhalb des Zeitintervalls des Plateaus anliegende Druckverlauf wird ausgewertet. Um hier auf die tatsächlichen Druckverhältnisse im Blutgefäß rückschließen zu können, wird die bei der Initialisierung ermittelte Übertragungsfunktion invertiert und auf die bei der Niedrigdruck-Plateaumessung ermittelten Druckverläufe im Sinne der Lösung eines inversen Problems angewendet.

Die einfache Lösung des inversen Problems, d.h. des Rückschlusses auf das Quellsignal von einer Beobachtung nach der Gleichung

$$P_{\text{brach}}(j\omega) = \frac{P_{\text{osc}}(j\omega)}{H_k(j\omega)}$$

ist für den stationären Fall möglich, wenn Übertragungsfunktion und Gegendruck bekannt und konstant sind.

Die modellbasierte Blutdruckbestimmung ermöglicht durch die Lösung eines Differentialgleichungssystems im Zeitbereich auch die Berücksichtigung von zeitlich veränderlichen Gegendrücken, d.h. von Messsignalen, die keine oder nur eine schwache Stationarität aufweisen.

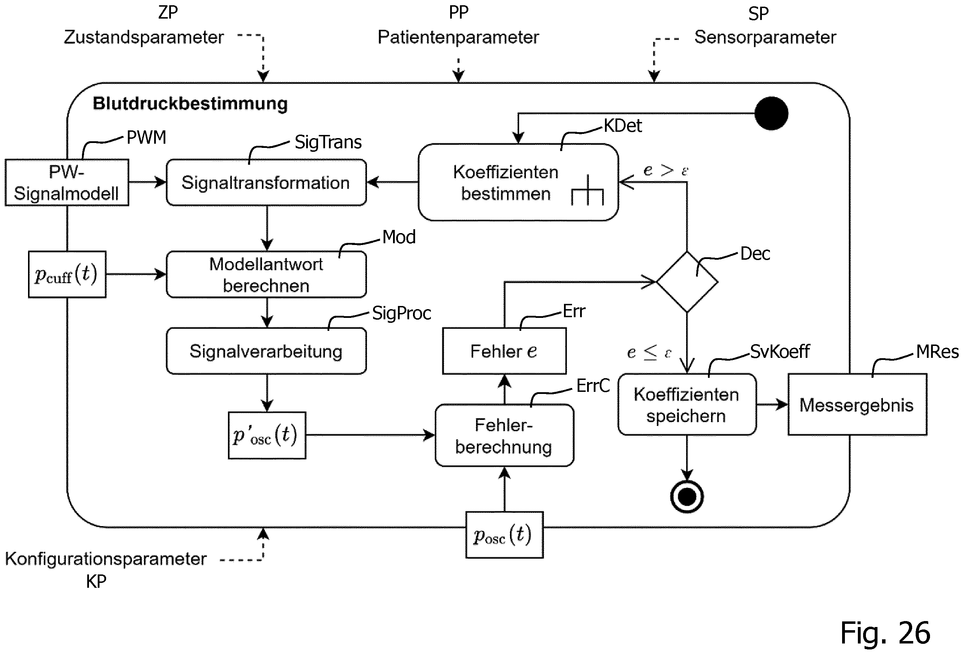

Fig. 26 zeigt, analog zur iterativen Koeffizientenbestimmung in der Initialisierung in Fig. 21, die Schritte zur modellbasierten Blutdruckbestimmung, bei der das PW-Signalmodell durch eine Signaltransformation (bspw. durch eine Skalierung) solange angepasst wird, bis die Abweichung zwischen dem berechneten oszillierenden Signalanteil $p'_{osc}(t)$ und dem aus der Niedrigdruck-Plateaumessung bestimmten oszillierenden Signalanteil $p_{osc}(t)$ minimal wird. Dabei wird wie in Fig. 24 aufgeführt die Modellantwort für das vorgegebene PW-Signal bestimmt. Der iterative Vorgang kann in einen globalen multivariaten Optimierer eingebunden werden, der eine Kostenfunktion minimiert. Die Kostenfunktion bildet dabei die Abweichung zwischen gemessenen und berechneten Signalanteil ab.

Fig. 26 zeigt ein Ablaufplan einer iterativen modellbasierten Blutdruckbestimmung, bei der ein PW-Signal aus dem PW-Signalmodell PWM mithilfe einer Signaltransformation SigTrans berechnet wird. Dieses PW-Signal bildet zusammen mit dem zeitvariablen Manschettendruck $p_{cuff}(t)$ die Eingangsvariablen für die Berechnung des in der Niedrigdruck-Plateaumessung erfassten oszillierenden Signalanteils $p_{osc}(t)$. Die Koeffizienten für die Signaltransformation des PW-Signalmodells werden durch einen Optimierer solange angepasst, bis der berechnete und gemessene Signalanteil übereinstimmen bzw. die Abweichung e eine vorgegebene Schwelle $\varepsilon$ erreicht oder unterschreitet. Aus den Koeffizienten (z.B. Offset der Pulswelle, Pulsdruck, PW-Formverzerrungsfaktoren) können anschließend die in der peripheren Arterie vorliegenden Blutdrücke berechnet werden.

Als Eingangsvariablen der Blutdruckbestimmung liegt somit ein Pulswellensignalmodell PWM, der zeitlich veränderliche Manschettendruck $p_{cuff}(t)$ sowie der oszillierende Signalanteil $p_{osc}(t)$ vor. Zusätzlich gehen Zustandsparameter ZP, Patientenparameter PP des Patienten oder Anwenders, Sensorparameter SP und Konfigurationsparameter KP in den Prozessablauf als Randbedingungen ein.

Die Daten des PW-Signalmodells PWSig werden über eine Signaltransformation SigTrans mit anfänglich vorliegenden Koeffizienten kombiniert. Der während der Messung vorliegende Manschettendruck $p_{cuff}(t)$, zusammen mit den aus der Signaltransformation resultierenden Daten dient zum Bestimmen einer Modellantwort Mod, die über eine Signalverarbeitung SigProc in einen modellierten ozillierenden Anteil $p'_{osc}(t)$ umgeformt wird. Dieser kann nun mit dem tatsächlich gemessenen oszillierenden Anteil $p_{osc}(t)$ verglichen werden. Es erfolgt hierzu eine Fehlerberechnung ErrC, der als Ausgabe Err einen Fehler e liefert. Dieser kann in einem Vergleich Dec mit einer vorgegebenen Fehlerschranke $\varepsilon$ verglichen werden. Ist die Abweichung zu groß werden die Koeffizienten des PW-Signalmodells in einem Schritt KDet neu gesetzt, ist die Abweichung innerhalb der gegebenen Toleranz, werden die Koeffizienten gespeichert. Diese bilden dann das Messergebnis MRes der Blutdruckbestimmung.

Es wird bei dem Ablaufplan gemäß Fig. 26 somit ein gegebenes PulswellenSignalmodell auf die gemessenen Pulswellen angepasst und diese Anpassung liefert dann einen Aufschluss über die gesuchten hämodynamischen Parameter.

Wie im Ablauf für ein exemplarisches Homecare Niedrigdruck-Blutdruck-Monitoring in Fig. 5 dargestellt, erfolgt eine Validierung der Initialisierung im Rahmen der patientenspezifischen Konfiguration der Blutdruckmessvorrichtung.

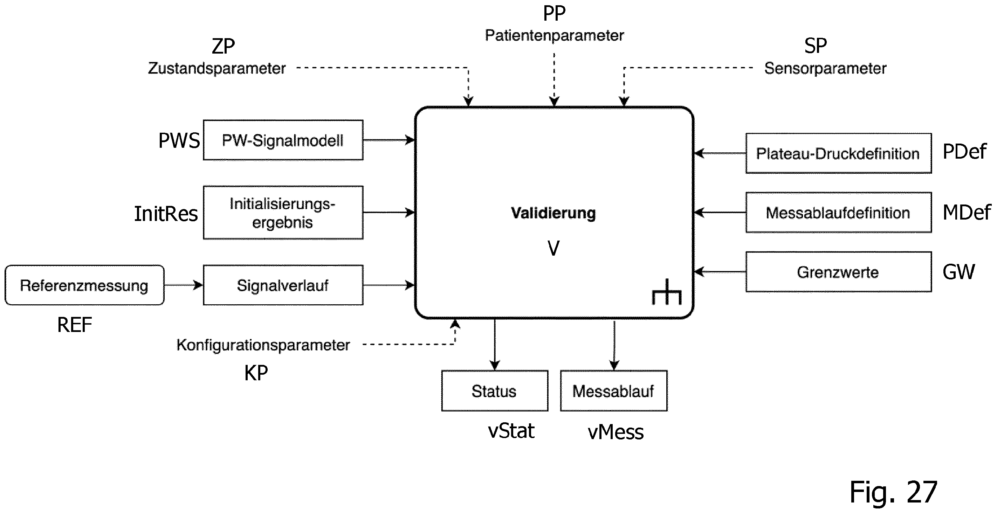

Die Ein- und Ausgangsparameter der Validierung sind schematisch in Fig. 27 dargestellt. Ziel der Validierung ist die Überprüfung des in der Initialisierung bestimmten personenspezifischen Übertragungsverhaltens sowohl für die in der Initialisierung verwendete Referenzmessung als auch für die im Messzeitraum des Niedrigdruck-Blutdruck-Monitorings zu verwendenden Niedrigdruck-Plateaumessungen.

Es erfolgt eine modellbasierte Blutdruckbestimmung gemäß den vorhergehend genannten Ausführungsbeispielen. Die einfachste Ausführung der Validierung ist die Blutdruckbestimmung anhand der Referenzmessdaten sowie die Durchführung von Niedrigdruck-Plateaumessungen für vorgegebene Plateau-Druckdefinitionen, Messablaufdefinitionen, z.B. wie viele Plateaus wie oft gemessen werden, und Grenzwerte. Aus den vorgegebenen Definitionen wird anschließend die Niedrigdruck-Plateaumessung mit der höchsten Übereinstimmung mit den Referenzwerten ausgewählt.

Bei der Wahl des Messablaufs mit den durchzuführenden Niedrigdruck-Plateaumessungen wird die Qualität und Ausprägung (ggf. die Klasse) der Pulswellen im Referenzsignal, die Qualität der Pulswellen der zugehörigen berechneten Modellantwort (p'$_{osc}$) sowie die aus der PW-Analyse bestimmten Pulswellenmerkmale berücksichtigt.

Als Eingangsparameter der Validierung V dienen zunächst die Zustandsparameter ZP, Patientenparameter PP des Patienten oder Anwenders, Sensorparameter PP und Konfigurationsparameter. Der messtechnische Input ist das PulswellenSignalmodell PWS, das Initialisierungsergebnis InitRes, schließlich eine Referenzmessung REF mit einem entsprechenden Signalverlauf, eine Plateau-Druckdefinition PDef, eine Messablaufdefinition MDef und Grenzwerte GW. Der Output der Validierung ist der validierte Status vStat und der validierte Messablauf vMess.

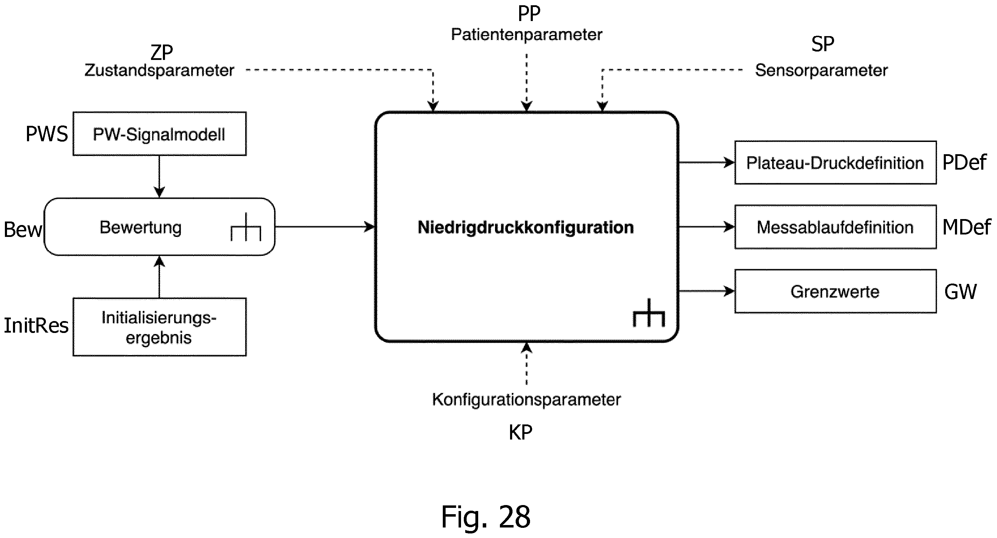

Eine erweiterte Ausführung der Validierung ist die Ableitung von Niedrigdruck-Plateaumessungen direkt aus dem Initialisierungsergebnis InitRes, bei der eine vorgegebene Anzahl von unterschiedlichen Niedrigdruckmessungen geplant wird, welche im Validierungsprozess durchgeführt und gegen die oszillometrische Referenzmessung sowie untereinander verglichen werden. Es wird entsprechend Fig. 28 eine Bewertung Bew des Initialisierungsergebnisses unter Berücksichtigung des personenspezifischen PW-Signalmodell PWS durchgeführt und eine individuelle Niedrigdruck-Konfiguration für den Patienten oder Anwender bestimmt, die die Plateau-Druckdefinition PDef, Messablaufdefinition MDef und Grenzwerte GW für die Blutdruckbestimmung enthält, die in der Validierung geprüft werden soll.

Bei einer oszillometrischen Messung mit der Bestimmung des oszillierenden Signalanteils aus einem vollständigen Ablassvorgang kann in einer weiteren Ausführungsvariante durch eine geeignete Signaltransformation eine Plateaurekonstruktion durchgeführt werden. Dies ermöglicht eine Blutdruckbestimmung mit einer virtuellen Niedrigdruck-Plateaumessung, bei der die Plateaumessung nicht physisch am Patienten oder Anwender durchgeführt wird, sondern von der Steuer- und Speichereinheit der Blutdruckmessvorrichtung simuliert wird. Dadurch kann die optimale Niedrigdruck-Konfiguration für den Patienten oder Anwender gefunden werden, ohne die Notwendigkeit einer Vielzahl von zusätzlichen Messungen. Die optimale Niedrigdruck-Konfiguration beinhaltet die Messparameter (z.B. konstanter Gegendruck) und die Anzahl der durchzuführenden Niedrigdruck-Plateaumessungen und werden im Messablauf gespeichert. Die von der Blutdruckmessvorrichtung durchgeführte Suche nach der optimalen Niedrigdruck-Konfiguration wird auch als dynamische [individuelle] Konfiguration bezeichnet und definiert den Messablauf für das Blutdruck-Monitoring.

Das Verfahren zum Betreiben einer Blutdruckmessvorrichtung erfolgt zusammengefasst also insbesondere mit folgenden Verfahrensschritten:

- Applizieren einer Messvorrichtung mit einer Druckmesseinheit und ggf. einer Druckeinheit an einer Messstelle am Körper.

- Initialisieren der Messvorrichtung mit einem Ausführen einer Referenzmessung und/oder durch ein Ausführen definierter Lageveränderungen der Messstelle und Bestimmen personen- und sensorspezifischer Parameter, die die Pulswellenübertragung der peripheren Arterie zum Messsensor charakterisieren, wobei folgende Schritte ausgeführt werden:

  a) Eine Abtastratenerhöhung durch Interpolation des vom Sensor erfassten zeitlichen Signalverlaufs.

  b) Eine Auftrennung des interpolierten Signalverlaufs in einen nichtoszillierenden und einen oszillierenden (pulsartigen) Signalanteil.

  c) Eine individuelle Artefakterkennung für die extrahierten Signalanteile.

  d) Eine Reduzierung und/oder ein Ausschluss der detektierten Artefakte in den extrahierten Signalanteilen.

  e) Eine Signalanalyse des pulsartigen Signalanteils zur Charakterisierung der Signaldynamik und Extraktion verschiedener Signalmerkmale, insbesondere der Fußpunkte der im pulsartigen Signalanteil auftretenden Pulswellen.

  f) Eine Ausrichtung des pulsartigen Signalanteils an detektierten Signalmerkmalen durch Interpolation des Signalverlaufs.

  g) Eine Zerlegung eines ausgerichteten, pulsartigen Signalanteils in Daten über Einzelperioden zum Identifizieren von einzelnen Pulswellen.

  h) Eine Klassifizierung und Qualitätsbewertung der einzelnen Pulswellen anhand der Pulswellenmorphologie und der individuellen Pulswellenmerkmale.

i) Eine Bildung einer Pulswellenvorlage aus einer geeigneten Teilmenge der klassifizierten Pulswellen und Pulswellenmerkmale durch eine Signaltransformation (mathematische Abbildungsvorschrift), um die Charakteristik der arteriellen Pulswellenform zu beschreiben.

j) Eine Rekonstruktion eines zum erfassten Referenzsignal zeitsynchronen Pulswellensignalmodells unter der Berücksichtigung der zeitvarianten Periodizität des pulsartigen Signalanteils des Referenzsignals.

k) Eine Rekonstruktion eines arteriellen Pulswellensignals durch Skalierung des Signalmodells mit den aus den Referenzmessung bekannten Blutdruckparametern.

l) Eine Ermittlung der individuellen Übertragungscharakteristik durch iterative Anpassung von personenspezifischen Parametern, die das rekonstruierte, skalierte Signalmodell möglichst genau auf das erfasste Referenzsignal abbildet.

- Speichern der personenspezifischen Initialisierungsparameter in einer Speicher- und Steuereinheit.

- Ausführen mindestens einer Blutdruckmessung bei Vorliegen eines Gegendrucks durch die Druckeinheit in einem subsystolischen Niedrigdruckbereich.

- Beibehalten des vorliegenden Gegendrucks innerhalb einer Plateauphase mit einer vorbestimmten Zeitdauer und Registrieren von zeitlichen Blutdruckverlaufsdaten während der vorbestimmten Zeitdauer.

- Umrechnen der registrierten zeitlichen Blutdruckverlaufsdaten über die Initialisierungsparameter in zeitliche arterielle Blutdruckdaten, wobei folgende Schritte ausgeführt werden:

a) Verarbeitungsschritte der Signalanalyse a) bis f) der Initialisierung, jedoch für die Blutdruckverlaufsdaten der im subsystolischen Niedrigdruckbereich erfassten Plateauphase.

b) Eine Rekonstruktion eines zu den erfassten Blutdruckverlaufsdaten zeitsynchronen Pulswellensignalmodell aus der in der Initialisierung erzeugten Pulswellenvorlage.

c) Eine Rekonstruktion eines arteriellen Pulswellensignals in der Steuer- und Speichereinheit mithilfe der individuellen Übertragungsparameter durch eine iterierte modellbasierte Blutdruckbestimmung, wobei über eine Signaltransformation eine abweichungsminimierende Anpassung von Parametern eines Pulswellensignalmodells an den oszillierenden Signalanteil des gemessenen Blutdruckverlaufsdaten erfolgt.

d) Eine Extraktion von Merkmalen aus dem berechneten, peripheren arteriellen Pulswellensignal.

e) Eine Auswertung der extrahierten Merkmale zum Ermitteln des peripheren Blutdrucks und weiterer hämodynamischer Parameter wie Pulswellengeschwindigkeit, Arterienalter, Herzzeitvolumen, etc.

- Reinitialisieren des Verfahrens, wenn die während des Messzeitraums ermittelte arterielle Pulswelle oder die damit assoziierten Blutdruckwerte oder hämodynamischen Parameter die in der Initialisierung festgelegten Grenzwerte über- oder unterschreiten.

Das genannte Verfahren wird zum Ermitteln und Analysieren von Pulswellen, zum Bestimmen eines Blutdrucks und weiterer hämodynamischer Parameter verwendet. Dabei werden nach der Initialisierung die dazu notwendigen Blutdruckverlaufsdaten im für den Patienten bzw. Anwender belastungsfreien subsystolischen Niedrigdruckbereich durchgeführt.

Der Gegenstand der Erfindung wurde anhand beispielhafter Ausführungsformen erläutert. Weitere Ausführungsformen ergeben sich außerdem aus den Unteransprüchen. Im Rahmen fachmännischen Handelns sind weitere Ausgestaltungen möglich.

Bezugszeichenliste

| | |
|---|---|
| 1 | Externe Auswerteeinheit |
| 10 | Anzeige |
| 11 | Konfigurationsprogramm |
| 12 | Auswerteprogramm |
| 13 | Benutzerschnittstelle |
| 14 | Geräteschnittstelle |
| 2 | Steuer- und Speichereinheit |
| 20 | Anzeige |
| 21 | Initialisierungsprogramm |
| 22 | Steuerprogramm |
| 23 | Speicher |
| 24 | Prozessor mit Bus |
| 25 | Digitaler Signalverarbeitungsprozessor |
| 27 | Schnittstelle |
| 29 | Schnittstelle zu externer Auswerteeinheit |
| 3 | Messeinheit, Initialisierungs- und Messeinheit |
| 30 | Befestigungsvorrichtung |
| 31 | Sensor |
| 32 | Aktuator |

| 33 | Puffer |
| 34 | Schnittstelle |
| 4 | Schnittstellenverbindung |
| 5 | Schnittstellenverbindung |
| 6 | Proband |
| | |
| A | Anzeigen der Messwerte |
| AbbV | Abbildungsvorschrift |
| Au | Aufpumpvorgang |
| AufInt | Aufpumpintervall |
| AbInt | Ablassintervall |
| Ab | Abpumpvorgang |
| Dec | Vergleichsschritt |
| DGLtest | Prüfen der ermittelten Lösung |
| DGSet | Setzen Anfangswerte Differentialgleichungssystem |
| e | Fehler |
| EpM | Einzelpunkt-Niedrigdruck-Plateaumessung |
| Err | Ausgabe Fehler |
| ErrC | Fehlerberechnung |
| EScalc | Berechnung Eingangssignal |
| FP | Fußpunkt |
| FPAdj | Fußpunktausrichtung |
| GW | Grenzwert |
| I | Initialisierung |
| InitRes | Initialisierungsergebnis |
| KDet | Koeffizientenbestimmung |
| KP | Konfigurationsparameter |
| M | oszillometrische Messung |
| Max | Maximum |
| Min | Minimum |
| Md | Speichern Modellantwort |
| MDef | Messablaufdefinition |
| MEx | Merkmalsextraktion |
| MP | medizinisches Personal |
| MZu | Merkmalszuordnung |
| pM | Plateaumessung |
| aG | aktive Gegendruckregelung |
| aS | aktiver Messsensor |
| pS | passiver Messsensor |
| PDef | Plateau-Druckdefinition |
| PP | Patientenparameter |
| PW | Pulswellen, extrahiert |
| axPW | approximiertes Pulswellensignal |
| PWA | Pulswellenanalyse |
| PWClas | Pulswellenklassifikation |
| PWEx | Pulswellenextraktion |
| PWM | Pulswellenmerkmale |
| PWMod | Pulswellen-Signalmodell |
| PWScal | Pulswellen-Skalierung |
| PWSig | Pulswellen-Signal |
| PWSlc | Pulswellen-Selektion |
| PWVo | Pulswellenvorlage |
| QB | Qualitätsbewertung |
| REF | Referenzmessung |
| ROI | Regions of Interest |
| S | Sensorprüfung |

| SigComp | Signalkomponenten |
| SigProc | Signalverarbeitung |
| SigTrans | Signaltransformation |
| SP | Sensorparameter |
| St | Auskultation |
| SvKoeff | Speicherung Koeffizienten |
| T | Tangentenverlauf |
| V | Validierung |
| vMess | validierter Messablauf |
| vStat | validierter Status |
| W | Wendepunkte |
| ZP | Zustandsparameter |

**Patentansprüche**

1. Verfahren zum Betreiben einer Blutdruckmessvorrichtung mit folgenden Verfahrensschritten:

   - Applizieren einer Messvorrichtung, enthaltend eine Druckmesseinheit und/oder eine Druckeinheit an einer Messstelle am Körper,
   - Initialisieren der Messvorrichtung mit einem Ausführen einer Referenzmessung und/oder durch ein Ausführen definierter Lageveränderungen der Messstelle und Bestimmen personenspezifischer Initialisierungsparameter,
   - Speichern der personenspezifischen Initialisierungsparameter in einer Speicher- und Steuereinheit,
   - Ausführen mindestens einer Blutdruckmessung bei Vorliegen eines Gegendrucks durch die Druckeinheit in einem subsystolischen Niedrigdruckbereich,
   - Beibehalten des vorliegenden Gegendrucks innerhalb einer Niedrigdruck-Plateauphase mit einer vorbestimmten Zeitdauer und Registrieren von zeitlichen Blutdruckverlaufsdaten während der vorbestimmten Zeitdauer,
   - Umrechnen der registrierten zeitlichen Blutdruckverlaufsdaten über die Initialisierungsparameter in zeitliche arterielle Blutdruckdaten, wobei.

   das Initialisieren durch Ausführen der Referenzmessung mit folgenden Schritten erfolgt:

   - Anlegen eines Messdrucks an die Messvorrichtung in einem definierten Druckbereich,
   - Definiertes Ändern, bspw. Ablassen, des Messdrucks aus der Messvorrichtung mit einem Registrieren des zeitlichen Druckverlaufs während des Vorgangs in der Steuer- und Speichereinheit,
   - Extraktion eines oszillierenden Pulsanteils aus dem zeitlichen Druckverlauf während des Vorgangs in Verbindung mit einer Speicherung einer Reihe von Daten zu einzelnen Pulswellen in der Steuer- und Speichereinheit,

- Signalanalyse der einzelnen Pulswellen und Datenabgleich mit einem gegebenen Pulswellensignalmodell durch die Steuer- und Speichereinheit,
- Ermitteln einer personenspezifischen Übertragungsfunktion aus dem Datenabgleich und Speichern der ermittelten Übertragungsfunktion als personenspezifischer Initialisierungsparameter in der Steuer- und Speichereinheit, wobei weiterhin nach der Initialisierung und der Bestimmung der Koeffizienten der Übertragungsfunktion mit einer oszillometrischen Niedrigdruck-Plateaumessung und der zum konstanten Plateau-Druck zugehörigen Übertragungsfunktion Hk(jω) aus einem oszillierenden Signalanteil der Niedrigdruck-Plateaumessung die arterielle Pulswelle berechnet wird und

die Signalanalyse der einzelnen Pulswellen mit folgenden Schritten erfolgt:

- Auswertung der Form der jeweiligen Pulswelle und Klassifizieren der jeweiligen Pulswelle in einer Auswerteeinheit und Ablegen in einem internen Speicher,
- Zusammenfügen und Transformieren von Pulswellen aus mindestens einer Klassifizierung zu mindestens einem, die Merkmale der arteriellen Pulswelle abbildenden Pulswellensignalmodell,
- Anpassen der gemessenen Pulswellenverläufe an das mindestens eine Pulswellensignalmodell und Ermitteln mindestens einer Übertragungsfunktion für das jeweilige Pulswellensignalmodell.

2. Verfahren nach Anspruch 1,
**dadurch gekennzeichnet, dass**
eine Validierung der Übertragungsfunktion ausgeführt wird, wobei bei der Blutdruckmessung bei einem gegebenen Gegendruck ermittelte und gespeicherte Blutdruckverlaufsdaten mit gegebenen Referenzparametern verglichen werden.

3. Verfahren nach Anspruch 1,
**dadurch gekennzeichnet, dass**
bei der Blutdruckmessung im subsystolischen Niedrigdruckbereich in der Steuer- und Speichereinheit eine Umrechnung der gemessenen zeitlichen Blutdruckwerte über eine invertierte, aus dem Initialisierungsschritt bestimmte personenspezifische Übertragungsfunktion in arterielle Blutdruckwerte erfolgt.

4. Verfahren nach Anspruch 1 oder 3,
**dadurch gekennzeichnet, dass**

bei der Blutdruckmessung im subsystolischen Niedrigdruckbereich in der Steuer- und Speichereinheit eine iterierte modellbasierte Blutdruckbestimmung ausgeführt wird, wobei über eine Signaltransformation eine abweichungsminimierende Anpassung von Parametern eines Pulswellensignalmodells an den oszillierenden Signalanteil des gemessenen Blutdrucks erfolgt.

5. Verfahren nach einem der vorhergehenden Ansprüche,
**dadurch gekennzeichnet, dass**
bei der Validierung der Initialisierungsparameter die modellbasierte Blutdruckbestimmung im subsystolischen Niedrigdruckbereich ausgeführt und mit dem Pulswellensignalmodell und einem Signalverlauf aus der Referenzmessung verglichen wird, wobei das dabei bestimmbare Initialisierungsergebnis mit den vorhandenen Initialisierungsparametern verglichen wird.

6. Verfahren nach Anspruch 1,
**dadurch gekennzeichnet, dass**
die Referenzmessung durch eine im Blutgefäß befindliche, z.B. katheterartige, Blutdruckmessvorrichtung erfolgt, wobei der dabei ermittelte Zeitverlauf des Blutdrucks mit einem parallel dazu ermittelten zeitlichen Druckverlauf an der Messvorrichtung abgeglichen und dabei die Übertragungsfunktion bestimmt wird.

7. Verfahren nach einem der vorhergehenden Ansprüche,
**dadurch gekennzeichnet, dass**
für die Initialisierung und/oder die Blutdruckmessung als Druckeinheit der Messvorrichtung eine aufpumpbare Druckmanschette oder eine Kombination aus einer Druckmanschette und einem einen konstanten subsystolischen Druck ausübenden Bekleidungsstück mit beliebiger Sensorbeschaffenheit verwendet wird.

8. Verfahren nach einem der vorhergehenden Ansprüche,
**dadurch gekennzeichnet, dass**
als Druckeinheit der Messvorrichtung eine aufpumpbare Druckmanschette in Kombination mit einem optischen oder elektromagnetischen Sensor verwendet wird, wobei die aufpumpbare Druckmanschette für das Ausführen der Referenzmessung und der Sensor für die Blutdruckmessung im subsystolischen Niedrigdruckbereich verwendet wird.

**Claims**

1. Method for operating a blood pressure measuring device with the following method steps:

- applying a measuring device, containing a pressure measuring unit and/or a pressure unit, to a measuring point on the body,
- initializing the measuring device by carrying out a reference measurement and/or by carrying out defined changes in position of the measuring point and determining person-specific initialization parameters,
- storing the person-specific initialization parameters in a memory and control unit,
- carrying out at least one blood pressure measurement in the presence of back pressure from the pressure unit in a subsystolic low-pressure range,
- maintaining the applied counterpressure within a low-pressure plateau phase of a predetermined duration and recording blood pressure data over time during the predetermined period,
- converting the recorded blood pressure data over time into arterial blood pressure data over time using the initialization parameters, wherein initialization is performed by carrying out the reference measurement with the following steps:

  - applying a measuring pressure to the measuring device in a defined pressure range,
  - defined changing, e.g. releasing, of the measuring pressure from the measuring device with a recording of the temporal pressure curve during the process in the control and storage unit,
  - extracting an oscillating pulse component from the pressure curve over time during the process in conjunction with storing a series of data on individual pulse waves in the control and storage unit,
  - signal analysis of the individual pulse waves and data matching with a given pulse wave signal model by the control and storage unit,
  - determining a person-specific transfer function from the data matching and storing the determined transfer function as person-specific initialization parameter in the control and storage unit, wherein, furthermore, after the initialization and the determination of the coefficients of the transfer function with an oscillometric low-pressure plateau measurement and the transfer function Hk(jω) associated with the constant plateau pressure from an oscillating signal portion of the low-pressure plateau measurement, the arterial pulse wave is calculated and

the signal analysis of the individual pulse waves is carried out with the following steps:

- evaluation of the shape of the respective pulse wave and classification of the respective pulse wave in an evaluation unit and storage in an internal memory,
- assembling and transforming pulse waves from at least one classification into at least one pulse wave signal model which represents the characteristics of the arterial pulse wave,
- adapting the measured pulse wave progressions to the at least one pulse wave signal model and determining at least one transfer function for the respective pulse wave signal model.

2.  Method according to claim 1, **characterized in that** a validation of the transfer function is carried out, wherein blood pressure profile data determined and stored during the blood pressure measurement at a given counterpressure are compared with given reference parameters.

3.  Method according to claim 1, **characterized in that** during the blood pressure measurement in the subsystolic low-pressure range, a conversion of the measured blood pressure values over time into arterial blood pressure values is carried out in the control and storage unit by means of an inverted, person-specific transfer function determined from the initialization step.

4.  Method according to claim 1 or 3, **characterized in that** during the blood pressure measurement in the subsystolic low-pressure range, an iterated model-based blood pressure determination is carried out in the control and storage unit, wherein a deviation-minimizing adaptation of parameters of a pulse wave signal model to the oscillating signal portion of the measured blood pressure is carried out by means of a signal transformation.

5.  Method according to one of the preceding claims, **characterized in that** during the validation of the initialization parameters, the model-based blood pressure determination is carried out in the subsystolic low-pressure range and is compared with the pulse wave signal model and a signal profile from the reference measurement, wherein the initialization result that can be determined in this way is compared with the existing initialization parameters.

6.  Method according to claim 1, **characterized in that** the reference measurement is carried out by means of a blood pressure measuring device situated in the blood vessel, for example a catheter-like blood pressure measuring device, wherein the blood pressure time curve determined in the process is compared with a pressure time curve determined in parallel at the measuring device and the transfer function is determined in the process.

**7.** Method according to one of the preceding claims, **characterized in that** an inflatable pressure cuff or a combination of a pressure cuff and a garment exerting a constant subsystolic pressure and having any sensor configuration is used as the pressure unit of the measuring device for the initialization and/or the blood pressure measurement.

**8.** Method according to one of the preceding claims, **characterized in that** an inflatable pressure cuff in combination with an optical or electromagnetic sensor is used as the pressure unit of the measuring device, wherein the inflatable pressure cuff is used for carrying out the reference measurement and the sensor for measuring blood pressure in the subsystolic low-pressure range.

**Revendications**

**1.** Procédé de fonctionnement d'un dispositif de mesure de la pression artérielle, comprenant les étapes suivantes consistant à :

- appliquer un dispositif de mesure, comprenant une unité de mesure de pression et/ou une unité de pression, à un point de mesure sur le corps,
- initialiser le dispositif de mesure en réalisant une mesure de référence et/ou en réalisant des changements de position définis du point de mesure, et déterminer des paramètres d'initialisation spécifiques à la personne,
- stocker les paramètres d'initialisation spécifiques à la personne dans une unité de stockage et de commande,
- réaliser au moins une mesure de la pression artérielle en présence d'une contre-pression par l'unité de pression dans une plage de basse pression sub-systolique,
- maintenir la contre-pression présente au sein d'une phase de plateau de basse pression pendant une durée prédéterminée, et enregistrer des données d'évolution temporelle de la pression artérielle pendant la durée prédéterminée,
- convertir les données enregistrées d'évolution temporelle de la pression artérielle en données temporelles de pression artérielle par le biais des paramètres d'initialisation,

l'initialisation par réalisation de la mesure de référence comprenant les étapes suivantes consistant à :

- appliquer une pression de mesure au dispositif de mesure dans une plage de pression définie,
- modifier de manière définie, par exemple abaisser, la pression de mesure du dispositif de mesure en enregistrant l'évolution temporelle de la pression dans l'unité de commande et de stockage pendant l'opération,
- extraire une composante impulsionnelle oscillante de l'évolution temporelle de la pression pendant l'opération, en association avec un stockage d'une série de données, relatives aux ondes de pouls individuelles, dans l'unité de commande et de stockage,
- analyser le signal des ondes de pouls individuelles et comparer les données avec un modèle donné du signal des ondes de pouls au moyen de l'unité de commande et de stockage,
- déterminer une fonction de transfert spécifique à la personne à partir de la comparaison des données, et stocker dans l'unité de commande et de stockage la fonction de transfert déterminée en tant que paramètre d'initialisation spécifique à la personne, et, après l'initialisation et la détermination des coefficients de la fonction de transfert, par une mesure oscillométrique du plateau basse pression et au moyen de la fonction de transfert $Hk(j\omega)$ associée à la pression constante du plateau, on calcule l'onde de pouls artérielle à partir d'une composante de signal oscillante de la mesure du plateau basse pression, et

l'analyse du signal des ondes de pouls individuelles est réalisée par les étapes suivantes consistant à :

- évaluer la forme de l'onde de pouls respective et classifier l'onde de pouls respective dans une unité d'évaluation, et l'enregistrer dans une mémoire interne,
- réunir et transformer des ondes de pouls d'au moins une classification en au moins un modèle de signal des ondes de pouls qui reproduit les caractéristiques de l'onde de pouls artérielle,
- adapter les évolutions mesurées des ondes de pouls audit au moins un modèle de signal des ondes de pouls, et déterminer au moins une fonction de transfert pour le modèle de signal respectif des ondes de pouls.

2. Procédé selon la revendication 1, **caractérisé en ce que** l'on procède à une validation de la fonction de transfert, les données d'évolution de la pression artérielle, déterminées et stockées lors de la mesure de la pression artérielle pour une contre-pression donnée, étant comparées à des paramètres de référence donnés.

3. Procédé selon la revendication 1, **caractérisé en ce que**, lors de la mesure de la pression artérielle dans la plage de basse pression sub-systolique, l'unité de commande et de stockage procède à une conversion des valeurs temporelles mesurées de la pression artérielle en valeurs artérielles de la pression artérielle par l'intermédiaire d'une fonction de transfert spécifique à la personne, qui est déterminée à l'étape d'initialisation et est inversée.

4. Procédé selon la revendication 1 ou 3, **caractérisé en ce que**, lors de la mesure de la pression artérielle dans la plage de basse pression sub-systolique, l'unité de commande et de stockage procède à une détermination de la pression artérielle basée sur un modèle et itérée, sachant qu'une adaptation, minimisant les écarts, des paramètres d'un modèle de signal de l'onde de pouls à la composante de signal oscillante de la pression artérielle mesurée est effectuée par l'intermédiaire d'une transformation de signal.

5. Procédé selon l'une des revendications précédentes, **caractérisé en ce que**, lors de la validation des paramètres d'initialisation, on procède à la détermination de la pression artérielle, basée sur le modèle, dans la plage de basse pression sub-systolique, et on la compare au modèle de signal de l'onde de pouls et à une courbe de signal issue de la mesure de référence, sachant que le résultat d'initialisation qui peut être déterminé à cette occasion est comparé aux paramètres d'initialisation existants.

6. Procédé selon la revendication 1, **caractérisé en ce que** l'on réalise la mesure de référence par un dispositif de mesure de la pression artérielle, par exemple de type cathéter, qui se trouve dans le vaisseau sanguin, et on compare l'évolution temporelle de la pression artérielle ainsi déterminée à une évolution temporelle de la pression déterminée en parallèle sur le dispositif de mesure, tout en déterminant la fonction de transfert.

7. Procédé selon l'une des revendications précédentes, **caractérisé en ce que**, pour l'initialisation et/ou la mesure de la pression artérielle, on utilise comme unité de pression du dispositif de mesure un brassard gonflable ou une combinaison d'un brassard et d'un vêtement exerçant une pression sub-systolique constante, ayant un capteur de nature quelconque.

8. Procédé selon l'une des revendications précédentes, **caractérisé en ce que** l'on utilise comme unité de pression du dispositif de mesure un brassard gonflable en combinaison avec un capteur optique ou électromagnétique, le brassard gonflable étant utilisé pour réaliser la mesure de référence, et le capteur étant utilisé pour mesurer la pression artérielle dans la plage de basse pression sub-systolique.

Fig. 1

EP 4 171 369 B1

Fig. 2

EP 4 171 369 B1

$p_{\mathrm{cuff}}(t)$

$p_{\mathrm{brach}}(t) \longrightarrow$ **Manschette mit Drucksensor** $\longrightarrow p_{\mathrm{osc}}(t)$

$P_{\mathrm{brach}}(j\omega) \longrightarrow H(j\omega, P_{\mathrm{cuff}}) \longrightarrow P_{\mathrm{osc}}(j\omega)$

Fig. 3

EP 4 171 369 B1

Fig. 4

Ab      pM      pM      pM      Ab

| Ablassvorgang | Plateau |
|---|---|

| Plateau | ... | Plateau |
|---|---|---|

| Ablassvorgang |
|---|

| Initialisierung | Validierung |
|---|---|

Gütekriterien nicht erfüllt

I     V

**Patientenspezifische Konfiguration**

| Monitoring |
|---|

**Messzeitraum**

| Re-Initialisierung |
|---|

(optional)
**Funktionsprüfung**

ca. 2 – 5 min      > 12 h      ca. 2 – 3 min

EP 4 171 369 B1

Fig. 5

use case Manschettenbasiertes Blutdruck-Monitoring (Var. A2)

Mobiles Blutdruckmessgerät

I

Initialisierung

extension points:
Validierung

<<extend>>

V

Validierung

<<include>>

Au/Ab

Aufpump- u.
Ablassvorgang

6

Patient

M

Oszillometrische
Messung

pM
EpM

Niedrigdruck-
Plateaumessung

A

Anzeige Messwerte

S

Sensorprüfung

Fig. 6

**use case** Manschettenbasiertes Blutdruck-Monitoring (Var. B)

Mobiles Blutdruckmessgerät

V

Validierung

<<extend>>

I

Initialisierung

**extension points:**
Validierung

MP

Medizinisches
Personal

6

Patient

M

<<include>>

Oszillometrische
Messung

Au/Ab

Aufpump- u.
Ablassvorgang

St

A

Anzeige Messwerte

pM

Niedrigdruck-
Plateaumessung

<<extend>>

Auskultation

**Stethoskop**

Fig. 7

EP 4 171 369 B1

Fig. 8

**use case** Blutdruck-Monitoring mit aktivem Sensor (Var. C2)

Mobiles Blutdruckmessgerät

A — Anzeige Messwerte

M — Oszillometrische Messung

Au/Ab — Aufpump- u. Ablassvorgang

Niedrigdruck-Plateaumessung — pM

6 — Patient

I — Initialisierung
extension points:
Validierung

<<include>>

<<extend>> — Validierung — V

<<include>>

<<include>>

aS

<<include>>

Niedrigdruck-Plateaumessung — pM

Aktive Gegendruck-regelung — aG

Konfiguration setzen

**Aktiver Messsensor**

Fig. 9

EP 4 171 369 B1

SP
Sensorparameter

InitR

Initialisierungs-ergebnis

Initialisierung
I

PP
Patientenparameter

Konfigurationsparameter KP

ZP
Zustandsparameter

PWMod

SigAn

SigComp

SigProc

$p_{rec}$, $p_{osc}$

PW-Signalmodell

Signalanalyse

Signalkomponenten

Signalverarbeitung

Signalverlauf

REF

Referenzmessung

Fig. 10

Fig. 11

EP 4 171 369 B1

Fig. 12

Fig. 13

Fig. 14

EP 4 171 369 B1

Fig. 15

Fig. 16

Fig. 17

Fig. 18

Fig. 19

Fig. 20

EP 4 171 369 B1

Fig. 21

Fig. 22

EP 4 171 369 B1

EP 4 171 369 B1

Fig. 23

Fig. 24

Fig. 25

Fig. 26

ZP
Zustandsparameter

PP
Patientenparameter

SP
Sensorparameter

Blutdruckbestimmung

PWM

PW-Signalmodell

SigTrans

Signaltransformation

KDet

Koeffizienten bestimmen

$e > \varepsilon$

$p_{\mathrm{cuff}}(t)$

Mod

Modellantwort berechnen

Dec

SigProc

Signalverarbeitung

Err

Fehler $e$

$e \leq \varepsilon$

SvKoeff

Koeffizienten speichern

MRes

Messergebnis

$p'_{\mathrm{osc}}(t)$

ErrC

Fehler-berechnung

$p_{\mathrm{osc}}(t)$

Konfigurationsparameter
KP

EP 4 171 369 B1

Fig. 27

Fig. 28

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2018263513 A
- US 2003069507 A